# EUROPEAN PATENT APPLICATION

(11) **EP 1 688 480 A2**
(43) Date of publication of application: **09.08.2006**
(21) Application number: 04818252.1
(22) Date of filing: 10.11.2004
(51) Int. Cl.: C12N 1/00

(54) **NOVEL ACETOACETYL-CoA REDUCTASE AND PROCESS FOR PRODUCING OPTICALLY ACTIVE ALCOHOL**

(30) Priority: 11.11.2003 JP 2003380987
(71) Applicant: KANEKA CORPORATION, Osaka-shi, Osaka 530-8288 (JP)
(72) Inventor: KAWANO, Shigeru, c/o Kaneya Corp. Takasago Plant, Takasago-shi, Hyogo 676-8688 (JP); YASOHARA, Yoshihiko, Kaneya Corp. Takasago Plant, Takasago-shi, Hyogo 676-8688 (JP)
(74) Representative: HOFFMANN EITLE
(86) International application number: PCT/JP2004/016666
(87) International publication number: WO 2005/044973

(57) **Abstract**

An object of the present invention is to provide a simple and easy process for producing optically active alcohols, specifically, a (R)-3-hydroxypentanenitrile, optically active 3-hydroxybutanoic esters, and optically active 1-phenylethanol derivatives, and to provide a novel enzyme useful for producing the above optically active alcohols, particularly a (R)-3-hydroxypentanenitrile.

The present invention provides a novel acetoacetyl-CoA reductase capable of asymmetrically reducing a 3-ketopentanenitrile to produce a (R)-3-hydroxypentanenitrile having an optical purity of 99%e.e. or more; and a process for allowing the novel enzyme or a known acetoacetyl-CoA reductase to act on each of the 3-ketopentanenitrile, an acetoacetic ester, and a 1-phenylethanone derivative to produce a corresponding optically active alcohol.

## Description

### Technical Field

The present invention relates to a novel useful acetoacetyl-CoA reductase. The present invention also relates to a process for producing optically active alcohols, inter alia, a (R)-3-hydroxypentanenitrile, optically active 3-hydroxybutanoic esters, and optically active 1-phenylethanol derivatives, using the above-described enzyme or a known acetoacetyl-CoA reductase. Optically active alcohols such as a (R)-3-hydroxypentanenitrile, optically active 3-hydroxybutanoic esters, and optically active 1-phenylethanol derivatives are compounds useful as raw materials or intermediates for synthesizing various pharmaceuticals, pesticides, and the like.

### Background Art

Optically active 3-hydroxypentanenitriles, particularly a (R)-3-hydroxypentanenitrile, are highly useful as production raw materials or synthetic intermediates for various pharmaceuticals, pesticides, and the like. Thus, there has been strong need for the development of an industrially practicable process for producing the (R)-3-hydroxypentanenitrile with high optical purity in good yield.

As a process for producing a (R)-3-hydroxypentanenitrile has been heretofore known a process which involves asymmetrically hydrolyzing a racemic 3-acetoxypentanenitrile using *Pseudomonas cepacia* lipase to subject to optical resolution into the (R)-3-hydroxypentanenitrile and a (S)-3-acetoxypentanenitrile (See Journal of Organic Chemistry, 62, 9165 (1997)). This process provides the (R)-3-hydroxypentanenitrile having an optical purity of 90%e.e., but is not an economical production process because it uses the enzyme at an amount of as large as one-half the weight of the substrate and has a yield of as low as 29%.

In addition, the above document describes that a thiocrown ether (1,4,8,11-tetrathiacyclotetradecane) may be added in the above-mentioned asymmetric hydrolysis reaction to provide a (R)-3-hydroxypentanenitrile having an optical purity of 99%e.e. or more in a yield of 49%. However, this process is not suitable for industrial practice from the viewpoints of cost and safety because the amount of the thiocrown ether necessary for the reaction is as extremely large as one-tenth the weight of the substrate. Further, for the technique in this document the reaction products the (R)-3-hydroxypentanenitrile and a (S)-3-acetoxypentanenitrile have been separated using silica gel column chromatography, but this separation process is also impractical because of its complexity and not advantageous to adopt industrially.

Under these circumstances, the present inventors invented a process for producing an optically active 3-hydroxypentanenitrile by subjecting a 3-ketopentanenitrile to asymmetric reduction using an enzyme, and filed an application earlier (See WO03/031636). This process can provide, in an extremely simple and easy manner, a (R)-3-hydroxypentanenitrile having high optical purity (94.8%e.e.), but there has remained room for improvement therein to obtain the desired compound with higher optical purity.

The optically active 3-hydroxybutanoic esters, particularly an (R)-3-hydroxybutanoic ester, are also highly useful as production raw materials or synthetic intermediates for various pharmaceuticals, pesticides, and the like.
Processes for producing a (R)-3-hydroxybutanoic ester include production by the asymmetric reduction of an acetoacetic ester using a biocatalyst. For example, there are known a process using baker's yeast (see Enzyme and Microbial Technology, 31, 656 (2002)), a process using a microorganism such as *Geotrichum candidum* or *Aspergillus niger* (see Journal of the Chemical Society, Chemical Communication, 7, 461 (1984)), and a process using glycerol dehydrogenase derived from *Geotrichum candidum* (see Tetrahedron Lett., 29, 2453 (1988)). However, these processes are practically not sufficient in terms of substrate charge concentration therefor and the ratio of conversion of substrate into product, and a more efficient synthesis process has been looked forward to

The optically active 1-phenylethanol derivative is also highly useful as production raw material or synthetic intermediate for various pharmaceuticals, pesticides, and the like.
As a process for producing the optically active 1-phenylethanol derivative by enzymatic asymmetric reduction is disclosed a process which involves allowing a microorganism belonging to the genus *Ashbya* or the genus *Ogataea* or a treated product thereof to act on a 2-halo-1-(substituted phenyl)ethanone to provide an optically active 2-halo-1-(substituted phenyl)ethanol (see WO92/01804, Japanese Patent Laid-Open No. 11-215995, and WO03/00911). In addition, a similar process using recombinant *Escherichia coli* into which the gene of a phenylacetaldehyde reductase derived from *Corynebacterium* sp. ST-10 has been introduced is disclosed (Eur. J. Biochem., 269, 2394 (2002)). However, each of these processes is low in the substrate charge concentration and the ratio of conversion of substrate into product, and there has been therefore need for a more efficient synthesis process.

On the other hand, enzymes reducing acetoacetyl-CoA (acetoacetyl-CoA reductases) using NADH or NADPH as a coenzyme were isolated from various microorganisms and their properties have been reported (see FEMS Microbiol. Lett., 52, 259-264 (1988) and J. Microbiol. Biotechnol., 6, 425-431 (1996)); however, it has not been reported that the acetoacetyl-CoA reductase is allowed to act on a 3-ketopentanenitrile, an acetoacetic ester or a 1-phenylethanone derivative to produce a corresponding optically active alcohol.

Disclosure of the Invention

### Problems to be Solved by the Invention

An object of the present invention is to provide a novel acetoacetyl-CoA reductase highly useful for the production of optically active alcohols, inter alia, a (R)-3-hydroxypentanenitrile. A further object of the present invention is to provide an efficient process for producing optically active alcohols, inter alia, the (R)-3-hydroxypentanenitrile, (R)-3-hydroxybutanoic esters, and optically active 1-phenylethanol derivatives.

### Means for Solving the Problems

As the result of intensive studies, the present inventors have found that an acetoacetyl-CoA reductase surprisingly can asymmetrically reduce a 3-ketopentanenitrile, acetoacetic esters, and 1-phenylethanone derivatives to produce a (R)-3-hydroxypentanenitrile, (R)-3-hydroxybutanoic esters, and optically active 1-phenylethanol derivatives, respectively.
Also, the present inventors have found and successfully isolated a novel acetoacetyl-CoA reductase, which has not been previously reported, having the activity of asymmetrically reducing a 3-ketopentanenitrile to produce a (R)-3-hydroxypentanenitrile having an optical purity of 99%e.e. or more. In addition, the present inventors have isolated a DNA encoding the enzyme and also successfully created a recombinant vector and a transformant expressing a large amount of the enzyme, thereby accomplishing the present invention.

Thus, the present invention relates to a novel acetoacetyl-CoA reductase acting on a 3-ketopentanenitrile using NADPH or NADH as a coenzyme to produce a (R)-3-hydroxypentanenitrile having an optical purity of 99%e.e. or more. The present invention also relates to a DNA encoding the acetoacetyl-CoA reductase, a recombinant vector containing the DNA, and a transformant producing the acetoacetyl-CoA reductase in a large quantity.

In addition, the invention relates to a practical process for producing a (R)-3-hydroxypentanenitrile having an optical purity of 99%e.e. or more, using the transformant.
Further, the invention relates to a practical process for producing useful optically active alcohols such as a (R)-3-hydroxypentanenitrile, (R)-3-hydroxybutanoic esters, and optically active 1-phenylethanol derivatives, using the acetoacetyl-CoA reductase.

The present invention is described below in detail.
As used herein, "acetoacetyl-CoA reductase" includes all of the enzymes having the ability to reduce acetoacetyl-CoA (acetoacetyl-CoA reducing activity).
The acetoacetyl-CoA reducing activity may be determined, for example, by the following method.
The reaction of a reaction solution containing 0.25 mM acetoacetyl-CoA, 0.25 mM NADPH or NADH, and the enzyme in 100 mM phosphate buffer (pH 6.5) may be effected at 30°C to determine the reducing activity by a decrease in absorbance at a wavelength of 340 nm with NADPH or NADH consumption.

As used in the invention, a 3-ketopentanenitrile can be synthesized, for example, by a method described in WO94/21617. As a 3-ketopentanenitrile, 3-ketopentanenitrile alkali metal salts represented by following formula (9) :

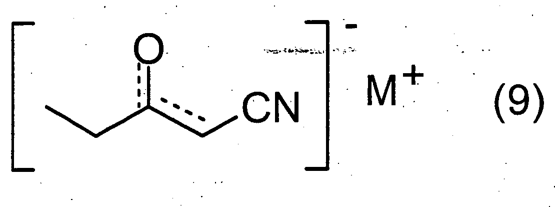

wherein M represents an alkali metal, such as, for example, a 3-ketopentanenitrile sodium salt, which can be obtained by a method described in WO03/031636, may be also used.

The novel acetoacetyl-CoA reductase which is one embodiment of the invention is first described in detail.
Examples of the acetoacetyl-CoA reductase of the invention include an acetoacetyl-CoA reductase having physicochemical properties shown in following (1) and (2) in which:
(1) the reductase acts, using NADPH or NADH as a coenzyme; on a 3-ketopentanenitrile represented by following formula (1):

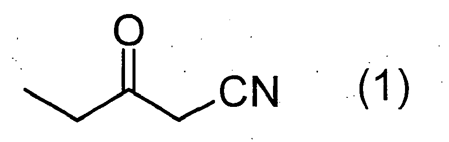

to produce a (R)-3-hydroxypentanenitrile represented by following formula (2):

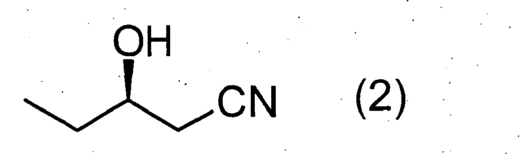

having an optical purity of 99%e.e. or more; and
(2) the reductase has a molecular weight of about 85,500 as determined by gel filtration analysis and about 26,000 as determined by SDS-polyacrylamide electrophoresis analysis.

The optical purity, the molecular weight by gel filtration analysis, and the molecular weight by SDS-polyacrylamide electrophoresis analysis may be determined as stated in Examples to be described.

Preferably, the acetoacetyl-CoA reductase further has physicochemical properties shown in following (3) to (5) in which:
(3) the reductase has an optimum temperature of 27 to 33°C;
(4) the reductase has an optimum pH of 5.5 to 6.5; and
(5) the reductase is inhibited by p-chloromercuribenzoic acid, copper sulfate, silver nitrate, or mercury chloride as an inhibitor.

Further examples of the acetoacetyl-CoA reductase of the invention can include (a) a polypeptide consisting of the amino acid sequence represented by SEQ ID NO: 1 of the Sequence Listing or (b) a polypeptide which consists of the amino acid sequence resulting from addition, deletion or substitution of one or more amino acid residues in the amino acid sequence represented by SEQ ID NO: 1 of the Sequence Listing and has the activity of acting on a 3-ketopentanenitrile to produce a (R)-3-hydroxypentanenitrile having an optical purity of 99%e.e. or more.

The polypeptide which consists of the amino acid sequence resulting from addition, deletion or substitution of one or more amino acid residues in the amino acid sequence represented by SEQ ID NO: 1 of the Sequence Listing can be prepared according to a well-known method as described e.g. in Current Protocols in Molecular Biology (John Wiley and Sons, Inc., 1989) and falls within the acetoacetyl-CoA reductase of the invention in so far as it has the activity of generating a (R)-3-hydroxypentanenitrile having an optical purity of 99%e.e. or more. In addition, since amino acid mutation may occur in nature, not only a polypeptide whose amino acid(s) has been artificially mutated but also a polypeptide whose amino acid(s) has been mutated in nature falls within the acetoacetyl-CoA reductase of the invention in so far as it has the activity of acting on a 3-ketopentanenitrile to produce the (R)-3-hydroxypentanenitrile having an optical purity of 99%e.e. or more.

Here, "the activity of acting on a 3-ketopentanenitrile to produce a (R)-3-hydroxypentanenitrile having an optical purity of 99%e.e. or more" may be confirmed under the following reaction and measurement conditions.
To a test tube in which 5 mg of a 3-ketopentanenitrile, 50 mg of NADPH, and 50 µl of 1 M phosphate buffer (pH 6.5) have been preliminarily placed is added 450 µl of a solution of the enzyme (or a cell-free extract), followed by shaking overnight at 30°C. The reaction solution is extracted with 1 ml of ethyl acetate, followed by determining the optical purity of the generated 3-hydroxypentanenitrile using capillary gas chromatography.

Capillary gas chromatography analysis conditions
Column: Chiradex G-TA (20 m x 0.25 mm) (from ASTEC Ltd.), Detection: FID
Column temperature: 120°C
Injection temperature: 200°C
Detection temperature: 200°C
Carrier gas: helium (100 kPa)
Split ratio: 100/1
Elution time: 4.37 minutes for a (R)-3-hydroxypentanenitrile, 5.17 minutes for a (S)-3-hydroxypentanenitrile.

The acetoacetyl-CoA reductase of the invention can be found from microorganisms, animals, or plants having the ability to convert a 3-ketopentanenitrile into a (R)-3-hydroxypentanenitrile. Methods for searching the acetoacetyl-CoA reductase of the invention include, for example, a method as described in WO03/031636.

For example, the search could be carried out from yeast as described below. In a test tube are placed 5 ml of a liquid culture medium (pH 7) consisting of 40 g of glucose, 3 g of yeast extract, 6.5 g of diammonium hydrogenphosphate, 1 g of potassium dihydrogenphosphate, 0.8 g of magnesium sulfate 7-hydrate, 60 mg of zinc sulfate 7-hydrate, 90 mg of iron sulfate 7-hydrate, 5 mg of copper sulfate 5-hydrate, 10 mg of manganese sulfate 4-hydrate, and 100 mg of sodium chloride (each per L) before sterilization, which is then inoculated with yeast, followed by shake culture at 30°C for 2 to 3 days. Subsequently, microbial cells are collected using centrifugation and suspended in 1 to 5 ml of a phosphate buffer containing 2 to 10% by weight of glucose, which is then added to a test tube in which 2.5 to 25 mg of a 3-ketopentanenitrile has been preliminarily placed, followed by shaking at 30°C for 2 to 3 days. This reaction may be conducted after disrupting the yeast. Also, NAD⁺ and/or NADP⁺ and glucose dehydrogenase and glucose or formate dehydrogenase and formic acid may be added. After conversion reaction, extraction could be carried out using an appropriate organic solvent, followed by analyzing the generated 3-hydroxypentanenitrile using e.g. capillary gas chromatography.

Origins of the acetoacetyl-CoA reductase of the invention can include, for example, a bacterium belonging to the genus *Achromobacter*, which is preferably *Achromobacter xylosoxidans* subsp. *denitrificans*, more preferably *Achromobacter xylosoxidans* subsp. *denitrificans* IFO15125 strain.

A process for obtaining the acetoacetyl-CoA reductase of the invention from microorganisms having the ability to asymmetrically reduce a 3-ketopentanenitrile to produce a (R)-3-hydroxypentanenitrile is described below, but the invention is not limited thereto.

A microorganism is cultured in an appropriate medium, and microbial cells are separated by centrifugation from the culture solution, followed by suspending the microbial cells in an appropriate buffer solution. The microbial cells can be disrupted or lysed using a physical means such as glass bead, a biochemical means such as enzyme, or the like, followed by further removing solid matter in the solution e.g. by centrifugation to provide a solution containing the enzyme of the invention. Alternatively, the enzyme solution can be directly obtained without separating the microbial cells from the culture solution, using a similar method to that mentioned above.

Further, purification techniques generally used by those skilled in the art, for example, ammonium sulfate precipitation, dialysis, and chromatography, may be also conducted alone or in combination. Examples of chromatography include hydrophobic interaction chromatography, ion exchange chromatography, and gel filtration chromatography. These can provide an enzyme solution containing no unnecessary proteins and thus having higher purity.

The DNA encoding the acetoacetyl-CoA reductase of the invention may be any DNA,encoding the above-described acetoacetyl-CoA reductase. For example, the DNA may be a DNA consisting of the base sequence represented by SEQ ID NO: 2 of the Sequence Listing.

A process for obtaining a DNA encoding the enzyme of the invention is described below, but the present invention is not limited thereto.
The purified enzyme is digested with endopeptidase before the cleaved fragments are purified with reversed-phase HPLC, followed by determining some of the partial amino acid sequences using a protein sequencer. Then, PCR (Polymerase Chain Reaction) primers are synthesized on the basis of the resultant partial amino acid sequences. Subsequently, from a microorganism representing the origin of the DNA, a chromosomal DNA of the microorganism is prepared using a conventional DNA isolation method, for example, the Hereford method (Cell, 18, 1261 (1979)). This chromosomal DNA is used as template to carry out PCR employing the above-mentioned PCR primers to amplify a portion (core sequence) of the DNA encoding the polypeptide, followed by determining the base sequence. The base sequence determination can be accomplished by the dideoxy chain termination method or the like, using, for e x amp le, an ABI 373A DNA Sequencer (from Applied Biosystems).

In order to reveal the base sequence of the peripheral region of the core sequence, the chromosomal DNA of the microorganism is then digested using a restriction enzyme whose recognition sequence is not present in the core sequence, followed by subjecting the generated DNA fragment to self cyclization using T4 ligase to prepare template DNA for inverse PCR (Nucleic Acids Res. 16, 8186 (1988)). On the basis of the core sequence, primers providing initiations of DNA synthesis toward the outside of the core sequence are then synthesized, and used to amplify the peripheral region of the core sequence by the inverse PCR. The base sequence of the DNA thus obtained can be revealed to determine the DNA sequence of the whole coding region of the acetoacetyl-CoA reductase of interest.

The vector used to introduce the DNA encoding the acetoacetyl-CoA reductase of the invention into a host microorganism and express the DNA in the host microorganism having it introduced may be any vector which enables the expression of the polypeptide gene in an appropriate host microorganism. Such vectors include, for example, a plasmid vector, a phage vector, and a cosmid vector. A shuttle vector, which enables genetic exchange with other host strains, can be also used. These vectors have a control element including an operatively linked promoter (lacUV5 promotor, trp promoter, trc promoter, tac promoter, lpp promoter, tufB promoter, recA promoter, pL promoter or the like), and can be suitably used as expression vectors containing an expression unit operatively liked to the DNA of the invention. For example, pUCNT (WO94/03613) and the like can be suitably used.

As used herein, the term "control element" refers to a base sequence having a functional promoter and any related transcription element (for example, an enhancer, a CCAAT box, a TATA box, and a SPI site).
As used herein, the term "operatively linked" refers to the linkage of DNA with various regulatory elements such as promoter and enhancer regulating the expression thereof in a state enabling them to operate in a host cell so as to express the gene. It is well known to those skilled in the art that the type and kind of the control element will vary depending on a host.

Host cells into which a vector containing the DNA of the invention is introduced include bacteria, yeast, filamentous fungi, plant cells, and animal cells, and bacteria, particularly *Escherichia coli,* are preferable from the view point of ease of introduction and expression efficiency.
The DNA of the invention may be introduced into the host cell by the law of the art. When *Escherichia coli* is used as a host cell, the DNA of the invention may be introduced, for example, by the calcium chloride method.

When a 3-ketopentanenitrile is asymmetrically reduced using the acetoacetyl-CoA reductase of the invention or a transformant having the ability to produce the reductase to synthesize a (R)-3-hydroxypentanenitrile having an optical purity of 99%e.e. or more, NADPH or NADH is required as a coenzyme. Although the required amount of NADPH or NADH can be added to the reaction system to accomplish the reaction, the reaction may be performed using an enzyme having the ability to convert the oxidized form of the coenzyme (NADP⁺ or NAD⁺) into the reduced form (NADPH or NADH) (hereinafter referred to as coenzyme-regenerating ability) together with the substrate thereof, that is, combining the coenzyme-regenerating system with the enzyme of the invention, to drastically reduce the used amount of the expensive coenzyme.

The enzyme having the coenzyme-regenerating ability may use hydrogenase, formate dehydrogenase, alcohol dehydrogenase, glucose-6-phosphate dehydrogenase, glucose dehydrogenase, or the like. Preferably, glucose dehydrogenase and formate dehydrogenase are used; glucose dehydrogenase is particularly preferable.
Such reaction can be also performed by adding the coenzyme-regenerating system into the asymmetrical reduction reaction system, but, when a transformant obtained by transformation employing both of the DNA encoding the enzyme of the invention and the DNA encoding glucose dehydrogenase is used as catalyst, the reaction may be efficiently conducted without adding, into the reaction system, the separately prepared enzyme having the coenzyme-regenerating ability.

Such a transformant is obtained by incorporating the DNA encoding the acetoacetyl-CoA reductase of the invention and the DNA encoding glucose dehydrogenase into a single vector and introducing it into a host cell, or is also obtained by incorporating these two kinds of DNA into an incompatible group of two different vectors, respectively and introducing them into an identical host cell.

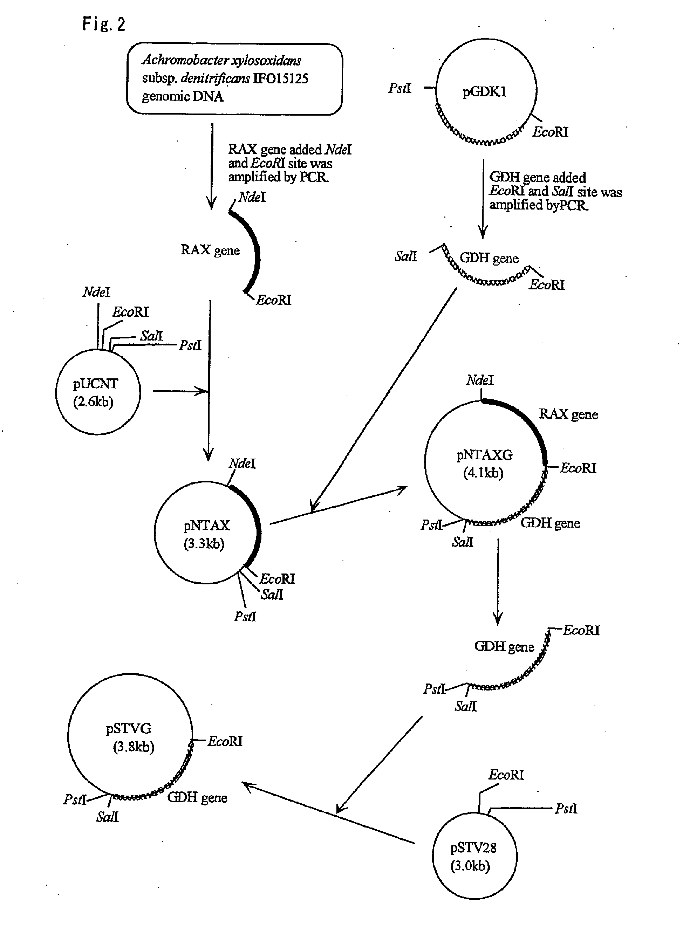

In this respect, the recombinant vector of the invention contains the DNA encoding the above-described acetoacetyl-CoA reductase or the DNA consisting of the base sequence represented by SEQ ID NO: 2 of the Sequence Listing, as above mentioned. Preferable examples of the vector include a recombinant vector represented by pNTAX in Figure 2 to be described.
Examples of the recombinant vector also include that further containing the DNA encoding the above-described glucose dehydrogenase. In this respect, the glucose dehydrogenase is preferably that derived from *Bacillus megaterium*.

The transformant of the invention is obtained by transforming a host cell using the above-described recombinant vector.
The transformant is preferably obtained by transforming a host cell using a first recombinant vector containing the DNA encoding the above-described acetoacetyl-CoA reductase or the DNA consisting of the base sequence represented by SEQ ID NO: 2 of the Sequence Listing, and a second recombinant vector containing the DNA encoding glucose dehydrogenase.
More preferable transformants include a transformant wherein the first recombinant vector is the above-described pNTAX and the above-described glucose dehydrogenase is derived from *Bacillus megaterium*, and a transformant wherein the first recombinant vector is the pNTAX and the second recombinant vector is a recombinant vector represented by pSTVG in Figure 2 to be described.
In this respect, the host cell is preferably *Escherichia coli.*

Specific examples of the transformant of the invention include a recombinant *Escherichia coli, E. coli* HB101 (pNTAX) (accession Number: FERM BP-10126, the original local deposit dated October 23, 2003has been transferred to the international deposit under the Budapest Treaty) and a recombinant *Escherichia coli, E. coli* HB101 (pNTAX, pSTVG) (accession number: FERM P-19567, deposit date: October 23, 2003), which have been deposited with International Patent Organism Depositary, National Institute of Advanced Industrial Science and Technology located at Central, 6, 1-1, Higashi 1-chome, Tsukuba-shi, Ibaraki-ken, Japan.

The process for producing a (R)-3-hydroxypentanenitrile by asymmetrically reducing a 3-ketopentanenitrile using the acetoacetyl-CoA reductase is then described in detail.

The acetoacetyl-CoA reductase used in the process may be employed without regard to the origin thereof, and may be that derived from an animal or a plant as well as a microorganism.

Examples thereof include known acetoacetyl-CoA reductases such as those derived from *Acinetobacter* sp. RA3849 (J. Bacteriol. ,177, 4501-4507 (1995)), *Ralstonia eutropha* (FEMS Microbiol. Lett., 52, 259-264 (1988)), *Alcaligenes latus* (J. Microbiol. Biotechnol., 6, 425-431 (1996)), *Azospirillum brasilense* (J. Gen. Microbiol., 136,1191-1196 (1990) and Mol. Gen. Genet., 231, 375-384 (1992)), *Azotobacter beijerinckii* (Biochem. J., 134, 225-238 (1973)), *Bacillus megaterium* (Can. J. Microbiol., 41 (Suppl.1), 77-79 (1995)), *Chromatium vinosum* D strain (Eur. J. Biochem., 209, 135-150 (1992)), *Ectothiorhodospira shaposhnikovii* (Appl. Microbiol. Biotechnol., 40, 292-300 (1993)), *Lupinus luteus* (Plant Soil, 56, 379-390 (1980)), *Methylobacterium extorquens* (FEMS Microbiol. Lett., 156, 275-279 (1997)), *Methylobacterium rhodesianum* MB126 (Arch. Microbiol., 161, 277-280 (1994)), *Paracoccus denitrificans* (FEMS Microbiol. Rev., 103, 257-264 (1992) and FEMS Microbiol. Lett., 133, 85-90 (1995)), *Rhizobium lupini* (Fiziol. Rast. (Moscow), 27, 544-550 (1980)), *Rhizobium meliloti* 41 (Microbiology, 141, 2553-2559 (1995)), *Rhodococcus ruber* NCIMB40126 (FEMS Microbiol. Rev., 103, 93-101 (1992)), *Synechococcus* sp. (Japanese Patent Laid-Open No. 08-187085), *Syntrophomonas wolfei* subsp. *wolfei* (Arch. Microbiol., 159, 16-20 (1993)), *Thiocapsa pfennigii* (Appl. Microbiol. Biotechnol., 40, 292-300 (1993)), and *Zoogloea ramigera* I-16-M (Arch. Microbiol., 114, 211-217 (1977) and J. Biol. Chem. 262, 97-102 (1987)).

Also, it can be the novel acetoacetyl-CoA-reductase of the invention described above in detail, which may be, for example, the polypeptide consisting of the amino acid sequence represented by SEQ ID NO: 1 of the Sequence Listing.

Further, examples of the acetoacetyl-CoA reductase used in the process include any of following (c) to (e):
(c) a polypeptide consisting of the amino acid sequence represented by SEQ ID NO: 3 of the Sequence Listing;
(d) a polypeptide consisting of the amino acid sequence resulting from addition, deletion or substitution of one or more amino acid residues in the amino acid sequence and having the activity of asymmetrically reducing a 3-ketopentanenitrile to produce a (R)-3-hydroxypentanenitrile having an optical purity of 95%e.e. or more; and
(e) a polypeptide encoded by a DNA hybridizing under stringent conditions to a DNA consisting of a base sequence complementary to the base sequence represented by SEQ ID NO: 4 of the Sequence Listing and having the activity of asymmetrically reducing a 3-ketopentanenitrile to produce a (R)-3-hydroxypentanenitrile having an optical purity of 95%e.e. or more.

The polypeptide is preferably derived from a microorganism belonging to the genus *Ralstonia*, particularly *Ralstonia eutoropha*.

A "DNA hybridizing under stringent conditions to a DNA consisting of a base sequence complementary to the base sequence represented by SEQ ID NO: 4 of the Sequence Listing" refers to a DNA obtained by using a colony hybridization, plaque hybridization, or southern hybridization method; or the like employing, as a probe, a DNA having a base sequence complementary to the whole base sequence represented by SEQ ID NO: 4 of the Sequence Listing. Specific examples include a DNA which can be identified by using a filter having a colony- or plaque-derived DNA fixed to perform hybridization at 65°C in the presence of 0.7 to 1.0 M NaCl and then washing the filter at 65°C employing an SSC solution with a 0.1 to 2 times higher concentration (an SSC solution with a one time concentration consists of 150 mM sodium chloride and 15 mM sodium citrate).

The hybridization may be carried out according to a method described, for example, in Molecular Cloning, A laboratory manual, second edition (Cold Spring Harbar Laboratory Press, 1989).

Specific examples of a hybridizable DNA include a DNA having a sequence identity to the base sequence represented by SEQ ID NO: 4 of the Sequence Listing of 60% or more, preferably 80% or more, more preferably 90% or more, even more preferably 95% or more, which may be used for the invention in so far as the encoded polypeptide has the activity of asymmetrically reducing a 3-ketopentanenitrile to produce a (R)-3-hydroxypentanenitrile having an optical purity of 95%e.e. or more.

Here, "sequence identity (%)" is represented by a numerical value obtained, when two DNAs to be compared are optimally aligned, by dividing, by the total number of bases to be compared, the number of the positions at which nucleic-acid bases (e.g. A, T, C, G, U, or I) correspond between both sequences and multiplying the result by 100.

The above-described acetoacetyl-CoA reductase can be allowed to act on a 3-ketopentanenitrile to provide a (R)-3-hydroxypentanenitrile having an optical purity of 95%e.e. or more. Particularly, use of the above-mentioned novel acetoacetyl-CoA reductase can provide the (R)-3-hydroxypentanenitrile with an extremely high optical purity of 99%e.e. or more.

In this respect, when the amino acid sequence of the above-described acetoacetyl-CoA reductase or the base sequence of the DNA encoding the enzyme is already determined, the same operation as that for the novel acetoacetyl-CoA reductase of the invention described above in detail may be conducted to prepare a transformant expressing a large amount of the enzyme, followed by performing reduction reaction using the transformant or the culture thereof.

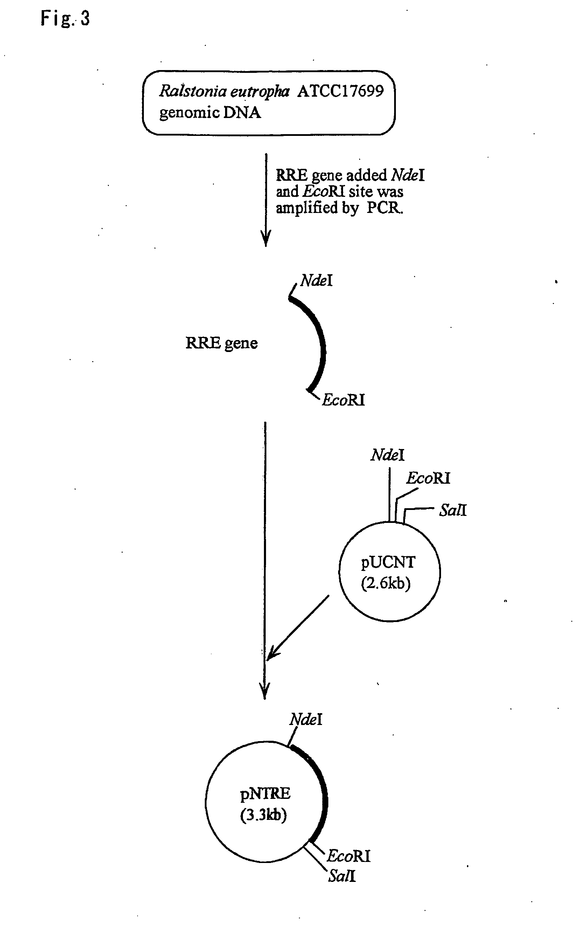

Here, a recombinant vector containing the DNA consisting of the base sequence represented by SEQ ID NO: 4 of the Sequence Listing and represented as pNTRE in Figure 3 to be described may be, for example, used in addition to the above-mentioned recombinant vectors.

Further, the following transformants may be, for example, used in addition to the above-mentioned transformants:
a recombinant *Escherichia coli, E. coli* HB101 (pNTRE) (accession number: FERM P-19566, deposit date: October 23, 2003); and
a recombinant *Escherichia coli, E. coli* HB101 (pNTRE, pSTVG) (accession number: FERM BP-10125, the original local deposit dated October 23, 2003 has been transferred to the international deposit under the Budapest Treaty) which have been deposited with International Patent Organism Depositary, National Institute of Advanced Industrial Science and Technology located at Central 6, 1-1, Higashi 1-chome, Tsukuba-shi, Ibaraki-Ken, Japan.

Reduction of a 3-ketopentanenitrile using a transformant expressing the acetoacetyl-CoA reductase to produce a (R)-3-hydroxypentanenitrile may be conducted as described below although it is not intended to be limited to the following process.

To an appropriate solvent is first added a 3-ketopentanenitrile or an alkali metal salt thereof (e.g., the 3-ketopentanenitrile sodium salt) as substrate, to which a coenzyme such as NADP⁺ and a culture product of the transformant are then added for reaction with stirring under the control of pH. This reaction is performed at a temperature of 5 to 80°C, preferably 10 to 60°C, more preferably 20 to 40°C, and, during reaction, the reaction solution is kept at a pH of 3 to 10, preferably 4 to 9, more preferably 5 to 8. The reaction may be conducted by a batch method or a continuous system.

In the case of the batch method, the reaction substrate can be added at a charge concentration of 0.01 to 70% (w/v), preferably 0.1 to 50%, more preferably 0.5 to 30%. When the substrate 3-ketopentanenitrile is added during the reaction, the unmodified 3-ketopentanenitrile or an alkali metal salt of the 3-ketopentanenitrile (e.g., the 3-ketopentanenitrile sodium salt) may be added.

The transformant may be cultured in a conventional nutrient liquid medium containing a carbon source, a nitrogen source, inorganic salts, organic nutrients, and the like in so far as the microorganism proliferates. The cultivation temperature is preferably 4 to 50°C.
Here, the culture product of the transformant may be not only a culture solution or microbial cells but also lyophilized microbial cells, acetone-dried microbial cells or a disrupted product thereof, a crude enzyme solution, or a purified enzyme, and further may be an immobilized enzyme or immobilized microbial cells immobilized using a well-known means.

When a transformant producing both the acetoacetyl-CoA reductase and the glucose dehydrogenase is used in performing the reaction, glucose can be further added to the reaction system to drastically reduce the used amount of the coenzyme.

A method for obtaining a (R)-3-hydroxypentanenitrile from the reaction solution is not particularly restricted, but high purity the (R)-3-hydroxypentanenitrile is easily obtained when it is extracted directly from the reaction solution or after separating microbial cells and the like, with a solvent such as ethyl acetate, toluene, t-butylmethyl ether, hexane, or methylene chloride, followed by dehydration and subsequent purification using silica gel column chromatography or the like.

The acetoacetyl-CoA reductase may be allowed to act on an acetoacetic ester represented by following general formula (3):

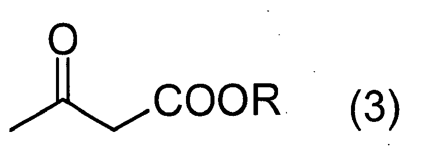

wherein R represents a lower alkyl group which is optionally substituted or branched to efficiently produce an (R)-3-hydroxybutanoic ester represented by following general formula (4):

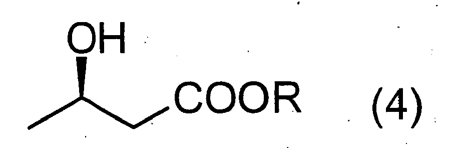

wherein R is the same as defined above.

Preferably, the lower alkyl group R is an optionally branched C1 to C5 alkyl group, more preferably methyl or ethyl. In addition, the lower alkyl group is optionally substituted, and examples of the substituent include a halogen atom, and hydroxyl, alkoxy, nitro, and amino groups.

The acetoacetyl-CoA reductase used in the reaction may be any such reductase without regard to the origin thereof in so far as it reduces an acetoacetic ester to an (R)-3-hydroxybutanoic ester; the novel acetoacetyl-CoA reductase of the invention and the above-described known acetoacetyl-CoA reductases may be suitably used. In addition, transformants expressing large amounts of these enzymes, which have been bred in the same way as the method described above, may be used. The reaction could be conducted in the same way as that for the reduction reaction of a 3-ketopentanenitrile described above.

An acetoacetyl-CoA reductase may be allowed to act on a 1-phenylethanone derivative represented by following formula (5):

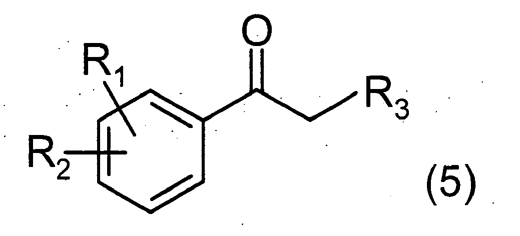

wherein R₁ and R₂ each represent a hydrogen atom, a halogen atom, an alkoxy group, or a nitro group, and may be the same or different; R₃ represents a hydrogen atom, a hydroxyl group, a halogen atom, or an alkyl group which may be optionally substituted, to efficiently produce an optically active 1-phenylethanol derivative represented by following formula (6) :

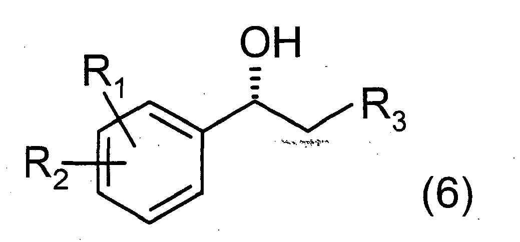

wherein R₁, R₂, and R₃ are each the same as defined above.
Particularly, an acetoacetyl-CoA reductase may be allowed to act on 2-chloro-1-(3'-chlorophenyl)ethanone represented by following formula (7):

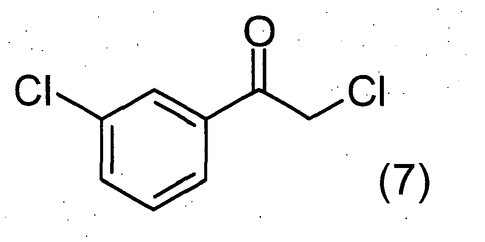

to efficiently produce (R)-2-chloro-1-(3'-chlorophenyl)ethanol represented by following formula (8):

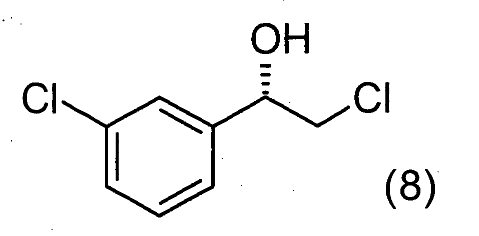

In R₁, R₂, and R₃, the halogen atom may be, for example, a fluorine atom, a chlorine atom, a bromine atom, or an iodine atom.
In R₁ and R₂, the alkoxy group is a C1 to C3 alkoxy group, including, for example, methoxy group, ethoxy group, or propoxy group. Methoxy group is preferable.
In R₃, the alkyl group is a C1 to C8 alkyl group, including, for example, methyl group, ethyl group, propyl group, hexyl group, or octyl group. A C1 to C2 alkyl is preferable. In this respect, the alkyl group is optionally substituted, and examples of the substituent include, for example, a fluorine atom, a chlorine atom, a bromine atom, a hydroxyl group, and an amino group.

The acetoacetyl-CoA reductase used in the reaction may be any such reductase without regard to the origin thereof in so far as it has the activity of reducing a 1-phenylethanone derivative to an optically active 1-phenylethanol derivative, particularly the activity of reducing 2-chloro-1-(3'-chlorophenyl)ethanone to (R)-2-chloro-1-(3'-chlorophenyl)ethanol; the novel acetoacetyl-CoA reductase of the invention and the above-described known acetoacetyl-CoA reductases may be suitably used. In addition, transformants expressing large amounts of these enzymes, which have been bred in the same way as the method described above, may be used. The reaction could be conducted in the same way as that for the reduction reaction of a 3-ketopentanenitrile described above.

### Advantages of the Invention

According to the invention, useful optically active alcohols such as a (R)-3-hydroxypentanenitrile, (R)-3-hydroxybutanoic esters, and optically active 1-phenylethanol derivatives may be produced in a simple and easy manner. Particularly for the (R)-3-hydroxypentanenitrile, non-conventional products having extremely high optical purities of 99.5%e.e. or more and, when the acetoacetyl-CoA reductase of the invention is used, 99%e.e. or more can be obtained

### Best Mode for Carrying Out the Invention

The present invention is described below in further detail with reference to Examples. However, this invention is not intended to be limited to these Examples in any manner. In the following description, "%" means "% by weight" unless otherwise noted.

The production amount of 3-hydroxypentanenitrile and the optical purity thereof were determined by analysis under the following capillary gas chromatography conditions.
Capillary gas chromatography analysis conditions
Column: Chiradex G-TA (20 m × 0.25 mm) (from ASTEC Ltd.), Detection: FID
Column temperature: 120°C
Injection temperature: 200°C
Detection temperature: 200°C
Carrier gas: helium (100 kPa)
Split ratio: 100/1
Elution time: 4.37 minutes for a (R)-3-hydroxypentanenitrile, 5.17 minutes for a (S)-3-hydroxypentanenitrile.

### Example 1: Purification of a novel acetoacetyl-CoA reductase from Achromobacter xylosoxidans subsp. denitrificans IFO15125 strain

The activity of reducing a 3-ketopentanenitrile to 3-hydroxypentanenitrile was determined as follows. To a test tube in which 5 mg of a 3-ketopentanenitrile and 30 mg of NADPH had been placed were added 0.05 ml of 1 M phosphate buffer (pH 6.5) and 0.45 ml of polypeptide (enzyme) solution, followed by shaking at 30°C for one hour. Thereto was added 1 ml of ethyl acetate, which was then thoroughly stirred, followed by centrifugation. The organic layer was analyzed under the above-described capillary gas chromatography conditions to determine the amount of 3-hydroxypentanenitrile produced. A reduction activity to produce 1 µmol of 3-hydroxypentanenitrile per minute under the conditions was defined as 1 unit. The optical purity of 3-hydroxypentanenitrile was also analyzed and determined under the capillary gas chromatography conditions.

Into a Sakaguchi flask was dispensed 100 ml of a liquid medium consisting of 10 g of meat extract, 10 g of peptone, 5 g of yeast extract, and 3 g of sodium chloride (each per L), followed by steam sterilization at 120°C for 20 minutes. Ten flasks of the liquid medium were provided, each of which then was aseptically inoculated with one platinum loop of *Achromobacter xylosoxidans* subsp. *denitrificans* IFO15125 strain, followed by shake culture at 30°C for 40 hours. Microbial cells were collected from 500 ml of the resultant culture solution by centrifugation, and washed with 200 ml of 10 mM phosphate buffer (pH 7.0), and the wet cells were suspended in 30 ml of a 10 mM phosphate buffer to which β-mercaptoethanol was added to give a concentration of 1 mM. Then, the microbial cells were disrupted using an ultrasonic homogenizer (from Branson), followed by removing the microbial cell residue using centrifugal separation to provide 25 ml of a cell-free extract. The extract was applied to a Toyopearl DEAE-650M (from Tosoh) column (10 ml) preliminarily equilibrated with a 20 mM phosphate buffer (pH 7.0) containing 1 mM β-mercaptoethanol to adsorb enzyme, followed by performing elution by increasing the concentration of sodium chloride stepwise (from 0 mM until 500 mM) to provide fractions having the activity of reducing a 3-ketopentanenitrile. Such active fractions were gathered, and applied to a HiLoad 16/60 Superdex 200 prep grade (from Amersham Pharmacia Biotech) column preliminarily equilibrated with a 20 mM phosphate buffer (pH 7.0) containing 1 mM β-mercaptoethanol. Further, the active fraction was applied to a 2'5'ADP Sepharose 4B (from Amersham Pharmacia Biotech) column (18 ml) preliminarily equilibrated with a 20 mM phosphate buffer (pH 7.0) containing 1 mM β-mercaptoethanol to adsorb enzyme, followed by eluting an active fraction with a linear gradient of sodium chloride (from 0 mM until 1.0 M). The above purification operation resulted in the provision of an electrophoretically almost single purified polypeptide preparation. In SDS polyacrylamide electrophoresis analysis, the molecular weight of a band was determined according to the protocol of an LMW calibration kit for SDS-electrophoresis from Amersham Bioscience. As a result, the molecular weight was approximately 26,000. By allowing this purified enzyme to act on a 3-ketopentanenitrile in the presence of NADPH, a (R)-3-hydroxypentanenitrile having an optical purity of 99.2%e.e. was produced. This acetoacetyl-CoA reductase is hereinafter referred to as "RAX".

### Example 2: Determining the properties of the enzyme

The enzymatic properties of the resultant RAX were examined.
(1) Action
   NADPH was used as a coenzyme to allow RAX to act on a 3-ketopentanenitrile to reduce it to a (R)-3-hydroxypentanenitrile having an optical purity of 99.2%e.e. When NADH was used as a coenzyme in place of NADPH to determine the 3-ketopentanenitrile-reducing activity thereof as described in Example 1, the activity was about 15% of that for the use of NADPH as a coenzyme.
   In addition, the activities of reducing acetoacetyl-CoA and ethyl 4-chloroacetoacetate were examined. A reaction solution containing 0.25 mM acetoacetyl-CoA or 10 mM ethyl 4-chloroacetoacetate, 0.25 mM NADPH, and the enzyme in a 100 mM phosphate buffer (pH 6.5) was subjected to reaction at 30°C to determine a reduction in absorbance at a wavelength of 340 nm with NADPH consumption to evaluate the activity of reducing each substrate. An enzyme activity for oxidizing 1 µmol of NADPH to NADP⁺ per minute under the conditions was defined as 1 unit. As a result, it was noted that RAX had the activities of reducing both acetoacetyl-CoA and ethyl 4-chrloroacetoacetate, which were about 7.6 times and about 3.2 times, respectively, the 3-ketopentanenitrile-reducing activity thereof determined as described in Example 1.

(2) Optimal temperature for action
To examine the optimal temperature for action of RAX, the ethyl 4-chloroacetoacetate reducing activity thereof was determined at 10 to 50°C. The activity was determined by adding 10 mM of the substrate ethyl 4-chloroacetoacetate, 0.25 mM of the coenzyme NADPH, and the enzyme to a 100 mM phosphate buffer (pH 6.5) for reaction at 10 to 50°C for 3 minutes and determining a reduction in absorbance at a wavelength of 340 nm. As a result, the optimal temperature was 27 to 33°C.

(3) Optimal pH for action
To examine the optimal pH for action of RAX, the ethyl 4-chloroacetoacetate reducing activity thereof was determined at a pH of 4 to 9. The enzyme activity was determined by adding 10 mM of the substrate ethyl 4-chloroacetoacetate, 0.25 mM of the coenzyme NADPH, and the enzyme to a buffer for reaction at 30°C for 3 minutes and determining a reduction in absorbance at a wavelength of 340 nm. The activity determination was performed at a pH range of 4 to 9 using a 100 mM phosphate buffer, a 100 mM acetate buffer, or a 100 mM Tris-hydrochloride buffer. As a result, the optimal pH was 5.5 to 6.5.

(4) Inhibitors
The ethyl 4-chloroacetoacetate reducing activity thereof was determined under the condition of adding any of various compounds and metal salts. The activity determination was performed by adding 10 mM of the substrate ethyl 4-chloroacetoacetate, 0.25 mM of the coenzyme NADPH, the enzyme, and an inhibitor to a 100 mM phosphate buffer (pH 6.5) for reaction at 30°C for 3 minutes and determining a reduction in absorbance at a wavelength of 340 nm. The activity in the absence of inhibitor is set to 100%, and the activities in the presence of inhibitors are defined as relative.activities in Table 1.

**Table 1**

| Inhibitor | Concentration (mM) | Relative activity |
|---|---|---|
| None | - | 100 |
| Iodoacetic acid | 1.0 | 98 |
| N-Ethylmaleimide | 1.0 | 103 |
| p-Chloromercuribenzoic acid | 1.0 | 0 |
| Phenylmethylsulfonyl fluoride | 1.0 | 108 |
| EDTA | 1.0 | 83 |
| MgSO₄ | 1.0 | 100 |
| MnCl₂ | 1.0 | 97 |
| ZnSO₄ | 1.0 | 81 |
| CoCl₂ | 1.0 | 108 |
| CuSO₄ | 1.0 | 0 |
| AgNO₃ | 0.1 | 0 |
| HgCl₂ | 0.01 | 0 |

As a result, the enzyme activity was inhibited by p-chloromercuribenzoic acid, copper sulfate, silver nitrate, or mercury chloride.
(5) Molecular weight
The gel filtration chromatography analysis of the purified enzyme by Superdex 200 HR 10/30 (from Pharmacia Biotech) was carried out using, as an eluting solution, a 50 mM phosphate buffer (pH 7.0) containing 150 mM sodium chloride. As a result, the molecular weight of the enzyme calculated from the relative retention time thereof with respect to a reference protein was approximately 85,500.

### Example 3: RAX gene cloning

The purified enzyme obtained in Example 1 was denatured in the presence of 8 M urea and then digested using an Achromobacter-derived lysyl endopeptidase (from Wako Pure Chemical Industries), followed by determining the amino acid sequences of the resultant peptide fragments by the Edman method.
In consideration of the DNA sequences predicted from the above amino acid sequences, primer 1 (5'-CARGGNTAYACNTTYTAYG-3': SEQ ID NO: 5) and primer 2 (5'-GCDATYTCYTCNGGNGTYCC-3': SEQ ID NO: 6) were synthesized. There was prepared 100 µl of a buffer for ExTaq containing 100 pmol each of the two kinds of primers (primers 1 and 2), 866 ng of a chromosomal DNA of *Achromobacter xylosoxidans* subsp. *denitrificans* IFO15125 strain, 10 nmol each of dNTP (deoxynucleotide triphosphate), and 2.5 U of ExTaq (from Takara Shuzo), and 30 cycles of thermal denaturation (96°C, 1 min.), annealing (50°C, 1 min.), and elongation reaction (72°C, 1 min.) were performed before cooling to 4°C, followed by identifying the amplified DNA using agarose gel electophoresis.

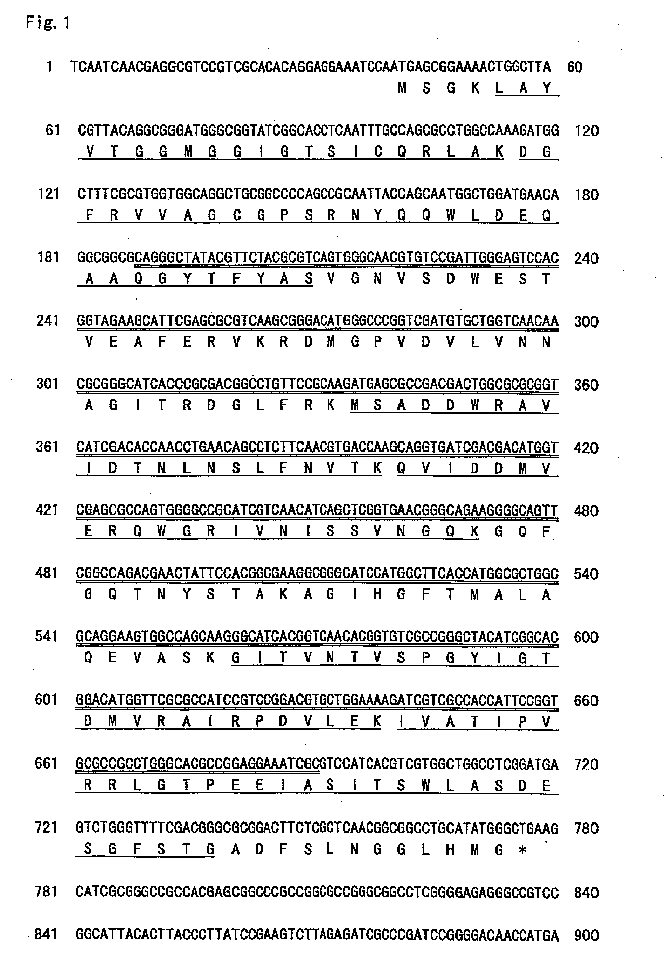

The chromosomal DNA of *Achromobacter xylosoxidans* subsp. *denitrificans* IFO15125 strain was prepared by the method for preparing a small amount of a bacterial genomic DNA as described in Bunshi Seibutsugaku Jikken (Molecular Biology Experiments) Protocol 1 (Maruzen Co., Ltd.), p.36. The amplified DNA was subcloned in the pT7Blue Vector (from Novagen) and subsequently sequenced. As a result, the amplified DNA, including the primer sequence, consisted of 503 bases. The DNA sequence is the doubly-underlined portion of the DNA sequence shown in Figure 1. This sequence is hereinafter referred to as "core sequence".

Based on a base sequence close to the 5' end of the core sequence, primer 3 (5'-CGTCGGCGCTCATCTTGCGGAACAG-3': SEQ ID NO: 7) providing a complementary sequence thereto was prepared; further, based on a base sequence close to the 3' end thereof, primer 4 (5'-AGGGCATCACGGTCAACACGGTGTC-3': SEQ ID NO: 8) was prepared. To prepare a template for inverse PCR, the chromosomal DNA of *Achromobacter xylosoxidans* subsp, *denitrificans* IFO15125 strain was first digested using the restriction enzyme SphI, and the digest was self-cyclized using T4DNA ligase. There was prepared 50 µl of a buffer for ExTaq containing 300 ng of the self-cyclized matter, 50 pmol each of the two kinds of primers (primers 3 and 4), 10 nmol each of dNTP, and 2.5 U of ExTaq (from Takara Shuzo), and 30 cycles of thermal denaturation (97°C, 0.5 min.), annealing (68°C, 1 min.), and elongation reaction (72°C, 5 min.) were performed before cooling to 4°C, followed by identifying the amplified DNA using agarose gel electophoresis.

The amplified DNA was subcloned in the pT7Blue Vector (from Novagen) and subsequently sequenced. From this result and the result of the core sequence, the whole base sequence of the gene encoding RAX was determined. The whole base sequence is shown in SEQ ID NO: 15 of the Sequence Listing. The whole base sequence and the deduced amino acid sequence encoded by the gene are defined in Figure 1. In Figure 1, the single underlines show the amino acid sequences determined by the Edman method in peptide fragments generated by subjecting the purified RAX to lysyl endopeptidase digestion.

### Example 4: Preparation of a recombinant vector containing the RAX gene

To express RAX in *Escherichia coli,* a recombinant vector used for transformation was prepared. A double-stranded DNA consisting of the RAX gene in which an NdeI site was added to the initiation codon region thereof and an additional termination codon and an EcoRI site were added immediately after the termination codon thereof was first obtained as described below.
Based on the base sequence determined in Example 3, primer 5 consisting of an initiation codon region of the RAX gene to which the NdeI site was added (5'-GTACATATGAGCGGAAAACTGGCTTACG-3': SEQ ID NO: 9) and primer 6, which had an additional termination codon and the EcoRI site added immediately after the termination codon of the RAX gene and, further, an NdeI site substituted by one base for destruction thereof, (5'-GTAGAATTCTTATCAGCCCATGTGCAGGCCGCCG-3': SEQ ID NO: 10) were synthesized. There was prepared 100 µl of a buffer for ExTaq containing 100 pmol each of the two kinds of primers (primers 5 and 6), 132 ng of a chromosomal DNA of *Achromobacter xylosoxidans* subsp. *denitrificans* IFO15125 strain, 20 nmol each of dNTP, and 2.5 U of ExTaq (from Takara Shuzo), and 30 cycles of thermal denaturation (97°C, 0.5 min.), annealing (65°C, 1 min.), and elongation reaction (72°C, 1 min.) were performed before cooling to 4°C, followed by identifying the amplified DNA using agarose gel electophoresis. This amplified fragment was digested with NdeI and EcoRI, and inserted into the NdeI and EcoRI sites downstream of the lac promoter of the plasmid pUCNT (WO94/03613) to provide a recombinant vector, pNTAX. The method for preparing pNTAX and the structure thereof are shown in Figure 2.

### Example 5: Preparation of a recombinant vector simultaneously containing both the RAX gene and the glucose dehydrogenase

A double-stranded DNA consisting of the gene of glucose dehydrogenase (hereinafter referred to as GDH) derived from *Bacillus megaterium* IAM1030 strain to which the Shine-Dalgarno sequence (9 nucleotides) of *Escherichia coli* 5 bases upstream of the initiation codon thereof, a EcoRI cleavage point immediately before the sequence, and an Sa1I cleavage point immediately after the termination codon thereof were added was obtained as described below.

Based on the base sequence information of the gene of GDH, primer 7, which had the Shine-Dalgarno sequence (9 nucleotides) of *Escherichia coli* added 5 bases upstream of the initiation codon of the structural gene of GDH and, further, the EcoRI cleavage point added immediately before the sequence, (5'-GCCGAATTCTAAGGAGGTTAACAATGTATAAAGATTTAGAAGG-3': SEQ ID NO: 11) and primer 8, which had the SalI site added immediately after the termination codon thereof, (5'-GCGGTCGACTTATCCGCGTCCTGCTTGG-3': SEQ ID NO: 12) were synthesized by the law of the art. Employing these two kids of primers, the plasmid pGDK1 (Eur. J. Biochem., 186, 389 (1989)) was used as a template to synthesize a double-stranded DNA by PCR. The resultant DNA fragment was digested with EcoRI and SalI, followed by inserting the digest into the EcoRI-SalI site of pNTAX constructed in Example 4 to provide a recombinant vector, pNTAXG. The method for preparing pNTAXG and the structure thereof are shown in Figure 2.

### Example 6: Preparation of a recombinant Escherichia coli producing RAX

The *Escherichia coli* HB101 (from Takara Shuzo) was transformed using the recombinant vector pNTAX obtained in Example 4 and the recombinant vector pNTAXG obtained in Example 5 to provide recombinant *Escherichia colis,* HB101 (pNTAX) and HB101 (pNTAXG), respectively. The recombinant *Escherichia coli HB101* (pNTAX) (Accession number: FERM BP-10126, the original local deposit dated October 23, 2003 has been transferred to the international deposit under the Budapest Treaty) has been deposited with International Patent Organism Depositary, National Institute of Advanced Industrial Science and Technology.

In addition, a DNA fragment of about 0.8 kb containing the GDH gene obtained by subjecting the recombinant vector pNTAXG prepared in Example 5 to double digestion using EcoRI and PstI was inserted into the EcoRI-PstI site of the plasmid pSTV28 (from Takara Shuzo) to construct a recombinant vector, pSTVG. The method for preparing pSTVG and the structure thereof are shown in Figure 2. The recombinant *Escherichia coli* HB101 (pNTAX), preliminarily made competent by the calcium chloride method, was transformed using this pSTVG to provide a recombinant *Escherichia coli*, HB101 (pNTAX, pSTVG). The recombinant *Escherichia coli* HB101 (pNTAX, pSTVG) as a transformant thus obtained (accession number: FERM P-19567, deposit date: October 23, 2003) has been deposited with International Patent Organism Depositary, National Institute of Advanced Industrial Science and Technology.

### Example 7: Expression of RAX and GDH in the recombinant Escherichia coli

Into a 500-ml Sakaguchi flask was dispensed 50 ml of 2xYT medium (pH 7) consisting of 1.6% (w/v) of Bacto tryptone, 1.0% (w/v) of Bacto yeast extract, and 10.5% (w/v) of NaCl, followed by steam sterilization at 120°C for 20 minutes. The recombinant *Escherichia colis* HB101 (pNTAX), HB101 (pNTAXG), and HB101 (pNTAX, pSTVG) obtained in Example 6, and a recombinant *Escherichia coli* HB101 (pUCNT), as a transformant containing only vector plasmid were each inoculated on the medium, followed by shake culture at 37°C for 18 hours. However, ampicillin was added so as to reach 120 µg/ml in culturing HB101 (pNTAX), HB101 (pNTAXG), or HB101 (pUCNT), and ampicillin and chloramphenicol were added so as to reach 120 µg/ml and 50 µg/ml, respectively in culturing HB101 (pNTAX, pSTVG). After collection, each of the bacteria was suspended in a 10 mM phosphate buffer (pH 7) and subjected to ultrasonic disruption to provide a cell-free extract. A 3-ketopentanenitrile-reducing specific activity and a GDH specific activity were determined for the cell-free extract from each recombinant *Escherichia coli*. The GDH specific activity was determined by adding 0.1 M of the substrate glucose, 2 mM of the coenzyme NADP⁺, and the enzyme to a 1M Tris hydrochloride buffer (pH 8.0) to determine an increase in absorbance at a wavelength of 340 nm at 25°C. An enzyme activity for reducing 1 µmol of NADP⁺ to NADPH per minute under the conditions was defined as 1 unit. The results are shown in Table 2.

**Table 2**

| Strain name | 3-ketopentanenitrile-reducing specific activity (units/mg) | GDH specific activity (units/mg) |
|---|---|---|
| HB101(pUCNT) | <0.1 | <0.01 |
| HB101(pNTAX) | 9.3 | <0.01 |
| HB101(pNTAXG) | 4.3 | 195 |
| HB101(pNTAX,pSTVG) | 7.0 | 10 |

The recombinant *Escherichia coli* HB101 (pNTAX) showed an apparent increase in a 3-ketopentanenitrile-reducing specific activity compared to that for the recombinant *Escherichia coli* HB101 (pUCNT) as a transformant containing only vector plasmid. The recombinant *Escherichia colis* HB101 (pNTAXG) and HB101 (pNTAX, pSTVG) showed apparent increases in the 3-ketopentanenitrile-reducing specific activity and GDH specific activity compared to those for the recombinant *Escherichia coli* HB101 (pUCNT) as a transformant containing only vector plasmid.

### Example 8: Synthesis of a (R)-3-hydroxypentanenitrile from a 3-ketopentanenitrile using the recombinant Escherichia coli HB101 (pNTAX)

The recombinant *Escherichia coli* HB101 (pNTAX) was cultured and subjected to cell disruption as described in Example 7 to prepare a cell-free extract. To a test tube in which 5 mg of a 3-ketopentanenitrile, 50 mg of NADPH, and 50 µl of 1 M phosphate buffer (pH 6.5) were preliminarily placed was added 450 µl of the above cell-free extract, followed by shaking overnight at 30°C. Extraction was then performed twice using 1 ml of ethyl acetate, followed by determining the production amount and optical purity of the resultant 3-hydroxypentanenitrile. As a result, the production amount was 3.7 mg, and the optical purity was (R)99.2%e.e.

### Example 9: Synthesis of a (R)-3-hydroxypentanenitrile from a 3-ketopentanenitrile using the recombinant Escherichia coli HB101 (pNTAXG)

The recombinant *Escherichia coli* HB101 (pNTAXG) was cultured and subjected to cell disruption as described in Example 7 to prepare a cell-free extract. To 450 µl of the cell-free extract were added 50 µl of 1 M phosphate buffer (pH 6.5), 13.9 mg of glucose, 1 mg of NADP⁺, and 5 mg of a 3-ketopentanenitrile, followed by stirring overnight at 30°C. After reaction, the production amount and optical purity of the resultant 3-hydroxypentanenitrile were determined. As a result, the production amount was 4.5 mg, and the optical purity was 99.2%e.e.

### Example 10: Synthesis of a (R)-3-hydroxypentanenitrile from a 3-ketopentanenitrile using the recombinant Escherichia coli HB101 (pNTAX, pSTVG)

The recombinant *Escherichia coli* HB101 (pNTAX, pSTVG) was cultured as described in Example 7. To 20 ml of the resultant culture solution were added 5.0 g of glucose, 3 mg of NADP⁺, and 0.2 g of a 3-ketopentanenitrile sodium salt, followed by stirring at 30°C while adding dropwise a 5N sodium hydroxide aqueous solution to adjust to pH 6.5. The addition of 0.2 g of the 3-ketopentanenitrile sodium salt was carried out 1.5, 3, 4.8, 5.7, 7, and 9 hours after the reaction. In addition, 4 ml of the culture solution and 3 mg of NADP⁺ were added 9.4 hours after the reaction. The production amount and optical purity of the resultant 3-hydroxypentanenitrile were determined 24 hours after the reaction. As a result, the production amount was 1.1 g, and the optical purity was 99.2%e.e.

### Example 11: Synthesis of a (R)-3-hydroxypentanenitrile from a 3-ketopentanenitrile using the recombinant Escherichia coli HB101 (pNTAX, pSTVG)

The recombinant *Escherichia coli* HB101 (pNTAX, pSTVG) was cultured as described in Example 7. In a 1-L separable flask were placed 400 ml of the resultant culture solution, 36.3 g of glucose, 50 mg of NADP⁺, and 3.5 g of a 3-ketopentanenitrile sodium salt, followed by adjusting pH to 6.5 before stirring at 30°C. A separately prepared 3-ketopentanenitrile aqueous solution having 18.5 g of the 3-ketopentanenitrile sodium salt dissolved in 37 ml of an ion exchanged water was added to maintain the pH of the reaction solution at 6.5. After adding the total amount of the 3-ketopentanenitrile aqueous solution, a 30% sodium hydroxide aqueous solution was added to keep the reaction solution at a pH of 6.5. After the end of reaction, the reaction solution was extracted with ethyl acetate, and the aqueous phase was further extracted with ethyl acetate. The organic phases were combined to remove the solvent under reduced pressure, followed by purification through distillation. As a result, 13.5 g of a (R)-3-hydroxypentanenitrile having an optical purity of 99.2%e.e. was obtained.

Example 12: Preparation of a recombinant vector containing the gene encoding an acetoacetyl-CoA reductase derived from *Ralstonia eutropha* ATCC17699 strain.
To express a *Ralstonia eutropha*-derived acetoacetyl-CoA reductase (hereinafter referred to as RRE) in *Escherichia coli,* a recombinant vector used for transformation was prepared. In obtaining the RRE gene, the base sequence information of the RRE gene described in J. Biol. Chem., 264, 15293 (1989) was consulted. A double-stranded DNA consisting of the RRE gene in which an NdeI site was added to the initiation codon region thereof and an additional termination codon and an EcoRI site were added immediately after the termination codon thereof was first obtained as described below.

Primer 9 consisting of an initiation codon region of the RRE gene to which the NdeI site was added (5'-AAGGAGTGCATATGACTCAGCGCATTGCGTATGTG-3': SEQ ID NO: 13) and primer 10, which had an additional termination codon and the EcoRI site added immediately after the termination codon thereof and, further, an NdeI site substituted by one base for destruction thereof, (5'-GTAGAATTCTTATCAGCCCATGTGCAGGCCGCCG-3': SEQ ID NO: 14) were synthesized. There was prepared 100 µl of a buffer for ExTaq containing 100 pmol each of the two kinds of primers (primers 9 and 10), 132 ng of a chromosomal DNA of *Ralstonia eutropha* ATCC17699 strain, 20 nmol each of dNTP, and 2.5 U of ExTaq (from Takara Shuzo), and 30 cycles of thermal denaturation (97°C, 0.5 min.), annealing (65°C, 1 min.), and elongation reaction (72°C, 1 min.) were performed before cooling to 4°C, followed by identifying the amplified DNA using agarose gel electophoresis. The chromosomal DNA of *Ralstonia eutropha* ATCC17699 strain used for PCR was prepared in the same way as that for the chromosomal DNA of *Achromobacter xylosoxidans* subsp. *denitrificans* IFO15125 strain as described in Example 3.
The above-described amplified fragment was digested with NdeI and EcoRI, and inserted into the NdeI-EcoRI site downstream of the lac promoter of the plasmid pUCNT (WO94/03613) to provide a recombinant vector, pNTRE. The method for preparing pNTRE and the structure thereof are shown in Figure 3.

### Example 13: Preparation of a recombinant Escherichia coli producing RRE

An *Escherichia coli* HB101 (from Takara Shuzo) was transformed using the recombinant vector pNTRE obtained in Example 12 to provide a recombinant *Escherichia coli* HB101 (pNTRE). The recombinant *Escherichia coli* HB101 (pNTRE) as a transformant thus obtained (accession number: FERM P-19566, deposit date: October 23, 2003) has been deposited with International Patent Organism Depositary, National Institute of Advanced Industrial Science and Technology.
The recombinant *Escherichia coli* HB101 (pNTRE), preliminarily made competent by the calcium chloride method, was transformed using the recombinant vector pSTVG obtained in Example 6 to provide a recombinant *Escherichia coli* HB101 (pNTRE, pSTVG). The recombinant *Escherichia coli* HB101 (pNTRE, pSTVG) as a transformant thus obtained (accession number: FERM BP-10125, the original local deposit dated October 23, 2003 has been transferred to the international deposit under the Budapest Treaty) has been deposited with International Patent Organism Depositary, National Institute of Advanced Industrial Science and Technology.

### Example 14: Expression of RRE and GDH in a recombinant Escherichia coli

The recombinant *Escherichia colis* HB101 (pNTRE) and HB101 (pNTRE, pSTVG) obtained in Example 13 and the recombinant *Escherichia coli* HB101 (pUCNT) as a transformant containing only vector plasmid were cultured as described in Example 7, followed by preparing a cell-free extract. A 3-ketopentanenitrile-reducing specific activity and a GDH specific activity were determined for the cell-free extract from each recombinant *Escherichia coli.* The results are shown in Table 3.

**Table 3**

| Strain name | 3-Ketopentanenitrile-reducing specific activity (units/mg) | GDH specific activity (units/mg) |
|---|---|---|
| HB101(pUCNT) | <0.1 | <0.01 |
| HB101(pNTRE) | 3.6 | <0.01 |
| HB101(pNTRE,pSTVG) | 2.9 | 19 |

The recombinant *Escherichia coli* HB101 (pNTRE) showed an apparent increase in a 3-ketopentanenitrile-reducing specific activity compared to that for the recombinant *Escherichia coli* HB101 (pUCNT) as a transformant *Escherichia coli* HB101 (pUCNT) as a transformant containing only vector plasmid. The recombinant *Escherichia coli* HB101 (pNTRE, pSTVG) showed apparent increases in the 3-ketopentanenitrile-reducing specific activity and GDH specific activity compared to those for the recombinant *Escherichia coli* HB101 (pUCNT) as a transformant containing only vector plasmid.

### Example 15: Synthesis of a (R)-3-hydroxypentanenitrile from a 3-ketopentanenitrile using the recombinant Escherichia coli HB101 (pNTRE)

The recombinant *Escherichia coli* HB101 (pNTRE) was cultured and subjected to cell disruption as described in Example 7 to prepare a cell-free extract. To a test tube in which 5.0 mg of a 3-ketopentanenitrile, 50 mg of NADPH, and 50 µl of 1 M phosphate buffer (pH 6.5) were preliminarily placed was added 450 µl of the above cell-free extract, followed by shaking overnight at 30°C. Extraction was then performed twice using 1 ml of ethyl acetate, followed by determining the production amount and optical purity of the resultant 3-hydroxypentanenitrile. As a result, the production amount was 2.5 mg, and the optical purity was (R)98.4%e.e.

### Example 16: Synthesis of a (R)-3-hydroxypentanenitrile from a 3-ketopentanenitrile using the recombinant Escherichia coli HB101 (pNTRE, pSTVG)

The recombinant *Escherichia coli* HB101 (pNTRE, pSTVG) was cultured as described in Example 7 to provide a culture solution. To 20 ml of the culture solution were added 5.0 g of glucose, 3 mg of NADP⁺, and 0.2 g of a 3-ketopentanenitrile sodium salt, followed by stirring at 30°C while adding dropwise a 5N sodium hydroxide aqueous solution to adjust to pH 6.5. The addition of 0.2 g of the 3-ketopentanenitrile sodium salt was carried out 2, 3, 5, 7, 8, and 9 hours after the reaction. In addition, 4 ml of the culture solution and 3 mg of NADP⁺ were added 10 hours after the reaction. The production amount and optical purity of the resultant 3-hydroxypentanenitrile were determined 24 hours after the reaction. As a result, the production amount was 1.0 g, and the optical purity was 98.3%e.e.

### Example 17: Synthesis of methyl (R)-3-hydroxybutanoate from methyl acetoacetate using the recombinant Escherichia coli HB101 (pNTAX, pSTVG)

The recombinant *Escherichia coli* HB101 (pNTAX, pSTVG) was cultured as described in Example 7. To 20 ml of the resultant culture solution were added 5.0 g of glucose, 3 mg of NADP⁺, and 0.4 g of methyl acetoacetate, followed by stirring at 30°C while adding dropwise a 5N sodium hydroxide aqueous solution to adjust to pH 6.5. The addition of 0.4 g of methyl acetoacetate was carried out 1.5, 3, 4.5, 5.5, and 7 hours after the reaction. The production amount of the resultant methyl 3-hydroxybutanoate was analyzed using capillary gas chromatography 24 hours after the reaction.
Analysis conditions
Column: TC-WAX (15 m x 0.25 mm) (from GL Science Inc.), Detection: FID, Column temperature: 85°C, Injection temperature: 200°C, Detection temperature: 200°C, Carrier gas: helium (70 kPa), Split ratio: 100/1, Elution time: 2.9 minutes for methyl acetoacetate, 3.8 minutes for methyl 3-hydroxybutanoate.

The methyl 3-hydroxybutanoate produced was dinitrobenzoylated before determining the optical purity thereof by HPLC analysis. The dinitrobenzoylation of methyl 3-hydroxybutanoate was carried out by extracting methyl 3-hydroxybutanoate with ethyl acetate from the reaction solution, and then adding 1.2 equivalents each of pyridine and 3,5-dinitrobenzoyl chloride based on the amount of methyl 3-hydroxybutanoate, followed by stirring at room temperature for 2 hours. After washing with 1N hydrochloride, purification was performed using thin layer chromatography for separation to provide the dinitrobenzoylated product of interest, which was then dissolved in ethanol and analyzed under the following HPLC analysis conditions.
Analysis conditions
Column: Chiralpak AD-H (from Daicel Chemical Industries, Ltd.), Detection wavelength: 230 nm, Column temperature: 20°C, Eluting solution: n-hexane/ethanol = 3/7, Flow rate: 0.7 ml/min., Elution time: 21.7 minutes for the S-form, 29.8 minutes for the R-form.
As a result, the production amount of methyl 3-hydroxybutanoate was 1.9 g, and the optical purity thereof was 97.2%e.e.

### Example 18: Synthesis of methyl (R)-3-hydroxybutanoate from methyl acetoacetate using the recombinant Escherichia coli HB101 (pNTRE, pSTVG)

A reaction was conducted as described in Example 17, except for use of the recombinant *Escherichia coli* HB101 (pNTRE, pSTVG) in place of the recombinant *Escherichia coli* HB101 (pNTAX, pSTVG), and, 24 hours after the reaction, the production amount and optical purity of the resultant methyl 3-hydroxybutanoate were determined. As a result, the production amount of methyl 3-hydroxybutanoate was 1.9 g, and the optical purity thereof was 98.3%e.e.

### Example 19: Synthesis of (R)-2-chloro-1-(3'-chlorophenyl)ethanol from 2-chloro-1-(3'-chlorophenyl)ethanone using the recombinant Escherichia coli HB101 (pNTAX, pSTVG)

The recombinant *Escherichia coli* HB101 (pNTAX, pSTVG) was cultured as described in Example 7. To 20 ml of the resultant culture solution were added 5.0 g of glucose, 3 mg of NADP⁺, and 0.4 g of 2-chloro-1-(3'-chlorophenyl)ethanone, followed by stirring at 30°C while adding dropwise a 5N sodium hydroxide aqueous solution to adjust to pH 6.5. The addition of 0.4 g of 2-chloro-1-(3'-chlorophenyl)ethanone was carried out 2, 4, 6, and 8 hours after the reaction. The production amount of the resultant 2-chloro-1-(3'-chlorophenyl)ethanol and the optical purity thereof were determined by analysis under the following conditions 24 hours after the reaction.

Analysis conditions
Column: Chiralpak OJ (from Daicel Chemical Industries, Ltd.), Detection wavelength: 210 nm, Column temperature: 20°C, Eluting solution: n-hexane/isopropanol = 39/1, Flow rate: 1.0 ml/min., Elution time: 25.3 minutes for 2-chloro-1-(3'-chlorophenyl)ethanone, 38.4 minutes for (R)-2-chloro-1-(3'-chlorophenyl)ethanol, 44.0 minutes for (S)-2-chloro-1-(3'-chlorophenyl)ethanol
As a result, the production amount of 2-chloro-1-(3'-chlorophenyl)ethanol was 1.9 g, and the optical purity thereof was 99.0%e.e.

### Example 20: Synthesis of (R)-2-chloro-1-(3'-chlorophenyl)ethanol from 2-chloro-1-(3'-chlorophenyl)ethanone using the recombinant Escherichia coli HB101 (pNTRE, pSTVG)

A reaction was conducted as described in Example 19, except for use of the recombinant *Escherichia coli* HB101 (pNTRE, pSTVG) in place of the recombinant *Escherichia coli* HB101 (pNTAX, pSTVG), and, 24 hours after the reaction, the production amount and optical purity of the resultant 2-chloro-1-(3'-chlorophenyl)ethanol were determined. As a result, the production amount of 2-chloro-1-(3'-chlorophenyl)ethanol was 1.9 g, and the optical purity thereof was 99.9%e.e.

### Industrial Applicability

According to the invention, useful optically active alcohols such as a (R)-3-hydroxypentanenitrile, (R)-3-hydroxybutanoic esters, and optically active 1-phenylethanol derivatives may be produced in a simple and easy manner. Particularly for a (R)-3-hydroxypentanenitrile, non-conventional products having extremely high optical purities of 95%e.e. or more and, when the acetoacetyl-CoA reductase of the invention is used, 99%e.e. or more can be obtained.

### Brief Description of the Drawings

Figure 1 is an illustration of the base sequence of the gene encoding RAX and the deduced amino acid sequence of RAX;
Figure 2 is a diagram showing the construction of recombinant vectors, pNTAX, pNTAXG, and pSTVG; and
Figure 3 is a diagram showing the construction of a recombinant vector, pNTRE.

## Claims

1. An acetoacetyl-CoA reductase having physicochemical properties shown in following (1) and (2) in which:
(1) the reductase acts, using NADPH or NADH as a coenzyme, on a 3-ketopentanenitrile represented by following formula (1): 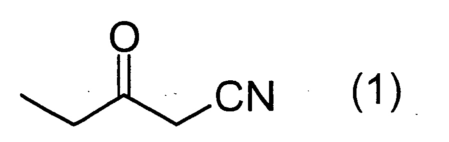 to produce a (R)-3-hydroxypentanenitrile represented by following formula (2): 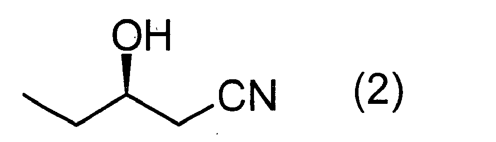 having an optical purity of 99%e.e. or more; and
(2) the reductase has a molecular weight of about 85,500 as determined by gel filtration analysis and about 26,000 as determined by SDS-polyacrylamide electrophoresis analysis.

2. The acetoacetyl-CoA reductase according to claim 1, further having physicochemical properties shown in following (3) to (5) in which:
(3) the reductase has an optimum temperature 27 to 33°C;
(4) the reductase has an optimum pH of 5.5 to 6.5; and
(5) the reductase is inhibited by p-chloromercuribenzoic acid, copper sulfate, silver nitrate, or mercury chloride as an inhibitor.

3. An acetoacetyl-CoA reductase which is a polypeptide described in following (a) or (b):
(a) a polypeptide consisting of an amino acid sequence represented by SEQ ID NO: 1 of the Sequence Listing; or
(b) a polypeptide which consists of the amino acid sequence resulting from addition, deletion or substitution of one or more amino acid residues in the amino acid sequence represented by SEQ ID NO: 1 of the Sequence Listing and has the activity of acting on a 3-ketopentanenitrile to produce a (R)-3-hydroxypentanenitrile having an optical purity of 99%e.e. or more.

4. The acetoacetyl-CoA reductase according to any one of claims 1 to 3 derived from a microorganism belonging to the genus *Achromobacter*.

5. The acetoacetyl-CoA reductase according to any one of claims 1 to 3 derived from a microorganism belonging to *Achromobacter xylosoxidans* subsp. *denitrificans*.

6. The acetoacetyl-CoA reductase according to any one of claims 1 to 3 derived from *Achromobacter xylosoxidans* subsp. *denitrificans* IFO15125 strain.

7. A DNA encoding the acetoacetyl-CoA reductase according to any one of claims 1 to 6.

8. A DNA consisting of a base sequence represented by SEQ ID NO: 2 of the Sequence Listing.

9. A recombinant vector comprising the DNA according to claim 7 or 8.

10. The recombinant vector according to claim 9 represented by pNTAX in Figure 2.

11. The recombinant vector according to claim 10 further comprising a DNA encoding a glucose dehydrogenase.

12. The recombinant vector according to claim 11, wherein the glucose dehydrogenase is derived from *Bacillus megaterium*.

13. A transformant obtained by transforming a host cell using the recombinant vector according to any one of claims 9 to 12.

14. A transformant obtained by transforming a host cell using a first recombinant vector comprising the DNA according to claim 7 or 8 and a second recombinant vector comprising a DNA encoding a glucose hydrogenase.

15. The transformant according to claim 14, wherein the first recombinant vector is pNTAX and the glucose hydrogenase is derived from *Bacillus megaterium*.

16. The transformant according to claim 14, wherein the first recombinant vector is pNTAX and the second recombinant vector is a recombinant vector represented by pSTVG in Figure 2.

17. The transformant according to any one of claims 13 to 16 wherein the host cell is *Escherichia coli.*

18. The transformant according to claim 17, wherein the transformant is *E.coli* HB101 (pNTAX) (FERM BP-10126).

19. The transformant according to claim 17, wherein the transformant is *E.coli* HB101 (pNTAX, pSTVG) (FERM P-19567).

20. A process for producing a (R)-3-hydroxypentanenitrile represented by following formula (2): 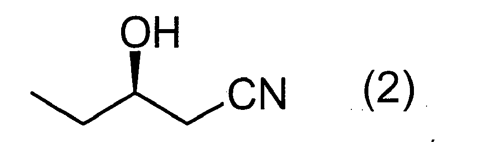 the process comprising allowing an acetoacetyl-CoA reductase to act on a 3-ketopentanenitrile represented by following formula (1) :

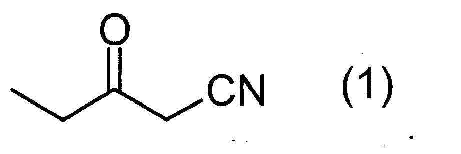

21. The production process according to claim 20 wherein the resultant (R)-3-hydroxypentanenitrile has an optical purity of 95%e.e. or more.

22. A process for producing an (R)-3-hydroxybutanoic ester represented by following formula (4): 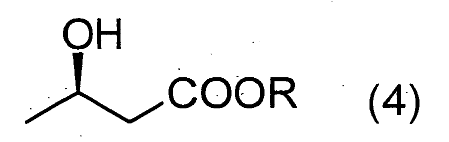 wherein R is a lower alkyl group which may be optionally substituted or branched , the process comprising allowing an acetoacetyl-CoA reductase to act on an acetoacetic ester represented by following formula (3): 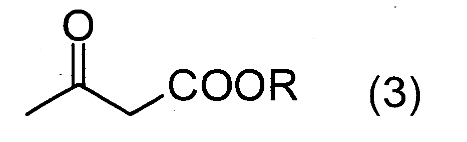 wherein R is the same as defined above.

23. A process for producing an optically active 1-phenylethanol derivative represented by following formula (6): 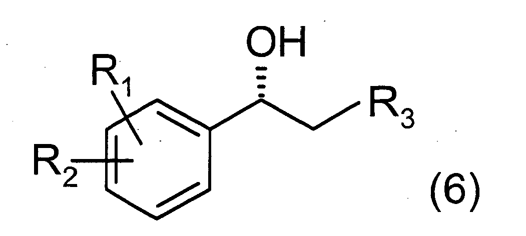 wherein R₁ and R₂ each represent a hydrogen atom, a halogen atom, an alkoxy group, or a nitro group, and may be the same or different respectively; and R₃ represents a hydrogen atom, a halogen atom, a hydroxyl group, or an alkyl group which may be optionally substituted, the process comprising allowing an acetoacetyl-CoA reductase to act on an 1-phenylethanone derivative represented by following formula (5): 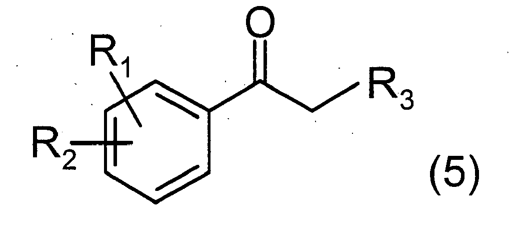 wherein R₁, R₂, and R₃ are each the same as defined above.

24. The production process according to claim 23, wherein the acetoacetyl-CoA reductase is allowed to act on 2-chloro-1-(3'-chlorophenyl)ethanone represented by following formula (7): 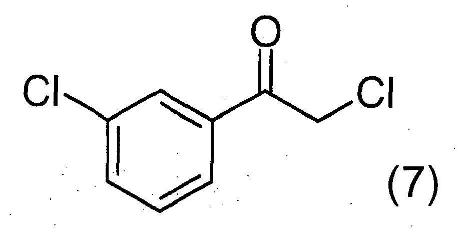 to produce (R)-2-chloro-1-(3'-chlorophenyl)ethanol represented by following formula (8):

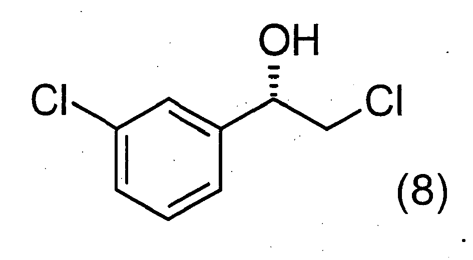

25. The production process according to any one of claims 20 to 24, wherein the acetoacetyl-CoA reductase is derived from a microorganism belonging to *Achromobacter*, *Acinetobacter, Ralstonia, Alcaligenes, Azospirillum, Azotobacter, Bacillus, Chromatium, Ectothiorhodospira, Lupinus, Methylobacterium, Paracoccus, Rhizobium, Rhodococcus, Synechococcus, Syntrophomonas, Thiocapsa,* or *Zoogloea*.

26. The production process according to any one of claims 20 to 24, wherein the acetoacetyl-CoA reductase according to any one of claims 1 to 6 is used.

27. The production process according to any one of claims 20 to 24, wherein the acetoacetyl-CoA reductase used is a culture product of the transformant according to any one of claims 13 to 19.

28. The production process according to any one of claims 20 to 24 wherein the acetoacetyl-CoA reductase used is a polypeptide described in any one of following (c) to (e):
(c) a polypeptide consisting of an amino acid sequence represented by SEQ ID NO: 3 of the Sequence Listing;
(d) a polypeptide consisting of the amino acid sequence resulting from addition, deletion or substitution of one or more amino acid residues in the amino acid sequence represented by SEQ ID NO: 3 of the Sequence Listing and having an activity of asymmetrically reducing a 3-ketopentanenitrile to produce a (R)-3-hydroxypentanenitrile having an optical purity of 95%e.e. or more; and
(e) a polypeptide encoded by a DNA hybridizing under stringent conditions to a DNA consisting of a base sequence complementary to a base sequence represented by SEQ ID NO: 4 of the Sequence Listing and having an activity of asymmetrically reducing a 3-ketopentanenitrile to produce a (R)-3-hydroxypentanenitrile having an optical purity of 95%e.e. or more.

29. The production process according to claim 28, wherein the acetoacetyl-CoA reductase is derived from a microorganism belonging to the genus *Ralstonia*.

30. The production process according to claim 28, wherein the acetoacetyl-CoA reductase is derived from a microorganism belonging to *Ralstonia eutoropha*.

31. The production process according to any one of claims 20 to 25, wherein a transformant expressing a glucose dehydrogenase is used in addition to the acetoacetyl-CoA reductase used in the production process according to any one of claims 28 to 30.

32. A recombinant vector comprising a DNA consisting of a base sequence represented by SEQ ID NO: 4 of the Sequence Listing and represented as pNTRE in Figure 3.

33. A transformant which is a recombinant *Escherichia coli, E.coli* HB101 (pNTRE) (FERM P-19566).

34. A transformant which is a recombinant *Escherichia coli, E.coli* HB101 (pNTRE, pSTVG) (FERM BP-10125).

35. The production process according to any one of claims 20 to 24 wherein the acetoacetyl-CoA reductase used is a culture product of the transformant according to claim 33 or 34.


SEQUENCE LISTING

<110> KANEKA CORPORATION

<120> NOVEL ACETOACETYL-CoA REDUCTASE AND PROCESS FOR PRODUCING OPTICALLY ACTIV
E ALCOHOL

<130> B030435W001

<150> JP2003-380987
<151> 2003-11-11

<160> 15

<170> PatentIn version 3.1

<210> 1
<211> 245
<212> PRT
<213> Achromobacter xylosoxidans subsp. denitrificans

<400> 1
Met Ser Gly Lys Leu Ala Tyr Val Thr Gly Gly Met Gly Gly Ile Gly
1               5                   10                  15

Thr Ser Ile Cys Gln Arg Leu Ala Lys Asp Gly Phe Arg Val Val Ala
            20                  25                  30

Gly Cys Gly Pro Ser Arg Asn Tyr Gln Gln Trp Leu Asp Glu Gln Ala
        35                  40                  45

Ala Gln Gly Tyr Thr Phe Tyr Ala Ser Val Gly Asn Val Ser Asp Trp
    50                  55                  60

Glu Ser Thr Val Glu Ala Phe Glu Arg Val Lys Arg Asp Met Gly Pro
65                  70                  75                  80

Val Asp Val Leu Val Asn Asn Ala Gly Ile Thr Arg Asp Gly Leu Phe
                85                  90                  95

Arg Lys Met Ser Ala Asp Asp Trp Arg Ala Val Ile Asp Thr Asn Leu
            100                 105                 110

Asn Ser Leu Phe Asn Val Thr Lys Gln Val Ile Asp Asp Met Val Glu
        115                 120                 125

Arg Gln Trp Gly Arg Ile Val Asn Ile Ser Ser Val Asn Gly Gln Lys
    130                 135                 140

Gly Gln Phe Gly Gln Thr Asn Tyr Ser Thr Ala Lys Ala Gly Ile His
145                 150                 155                 160

Gly Phe Thr Met Ala Leu Ala Gln Glu Val Ala Ser Lys Gly Ile Thr
                165                 170                 175

Val Asn Thr Val Ser Pro Gly Tyr Ile Gly Thr Asp Met Val Arg Ala
            180                 185                 190

Ile Arg Pro Asp Val Leu Glu Lys Ile Val Ala Thr Ile Pro Val Arg
        195                 200                 205

Arg Leu Gly Thr Pro Glu Glu Ile Ala Ser Ile Thr Ser Trp Leu Ala
    210                 215                 220

Ser Asp Glu Ser Gly Phe Ser Thr Gly Ala Asp Phe Ser Leu Asn Gly
225                 230                 235                 240

Gly Leu His Met Gly
                245


<210> 2
<211> 738
<212> DNA
<213> Achromobacter xylosoxidans subsp. denitrificans

<220>
<221> CDS
<222> (1)..(738)
<223>

<400> 2
atg agc gga aaa ctg gct tac gtt aca ggc ggg atg ggc ggt atc ggc 48
Met Ser Gly Lys Leu Ala Tyr Val Thr Gly Gly Met Gly Gly Ile Gly
1               5                   10                  15

acc tca att tgc cag cgc ctg gcc aaa gat ggc ttt cgc gtg gtg gca 96
Thr Ser Ile Cys Gln Arg Leu Ala Lys Asp Gly Phe Arg Val Val Ala
            20                  25                  30

ggc tgc ggc ccc agc cgc aat tac cag caa tgg ctg gat gaa cag gcg 144
Gly Cys Gly Pro Ser Arg Asn Tyr Gln Gln Trp Leu Asp Glu Gln Ala
        35                  40                  45

gcg cag ggc tat acg ttc tac gcg tca gtg ggc aac gtg tcc gat tgg 192
Ala Gln Gly Tyr Thr Phe Tyr Ala Ser Val Gly Asn Val Ser Asp Trp
    50                  55                  60

gag tcc acg gta gaa gca ttc gag cgc gtc aag cgg gac atg ggc ccg 240
Glu Ser Thr Val Glu Ala Phe Glu Arg Val Lys Arg Asp Met Gly Pro
65                  70                  75                  80

gtc gat gtg ctg gtc aac aac gcg ggc atc acc cgc gac ggc ctg ttc 288
Val Asp Val Leu Val Asn Asn Ala Gly Ile Thr Arg Asp Gly Leu Phe
                85                  90                  95

cgc aag atg agc gcc gac gac tgg cgc gcg gtc atc gac acc aac ctg 336
Arg Lys Met Ser Ala Asp Asp Trp Arg Ala Val Ile Asp Thr Asn Leu
            100                 105                 110

aac agc ctc ttc aac gtg acc aag cag gtg atc gac gac atg gtc gag 384
Asn Ser Leu Phe Asn Val Thr Lys Gln Val Ile Asp Asp Met Val Glu
        115                 120                 125

cgc cag tgg ggc cgc atc gtc aac atc agc tcg gtg aac ggg cag aag 432
Arg Gln Trp Gly Arg Ile Val Asn Ile Ser Ser Val Asn Gly Gln Lys
    130                 135                 140

ggg cag ttc ggc cag acg aac tat tcc acg gcg aag gcg ggc atc cat 480
Gly Gln Phe Gly Gln Thr Asn Tyr Ser Thr Ala Lys Ala Gly Ile His
145                 150                 155                 160

ggc ttc acc atg gcg ctg gcg cag gaa gtg gcc agc aag ggc atc acg 528
Gly Phe Thr Met Ala Leu Ala Gln Glu Val Ala Ser Lys Gly Ile Thr
                165                 170                 175

gtc aac acg gtg tcg ccg ggc tac atc ggc acg gac atg gtt cgc gcc 576
Val Asn Thr Val Ser Pro Gly Tyr Ile Gly Thr Asp Met Val Arg Ala
            180                 185                 190

atc cgt ccg gac gtg ctg gaa aag atc gtc gcc acc att ccg gtg cgc 624
Ile Arg Pro Asp Val Leu Glu Lys Ile Val Ala Thr Ile Pro Val Arg
        195                 200                 205

cgc ctg ggc acg ccg gag gaa atc gcg tcc atc acg tcg tgg ctg gcc 672
Arg Leu Gly Thr Pro Glu Glu Ile Ala Ser Ile Thr Ser Trp Leu Ala
    210                 215                 220

tcg gat gag tct ggg ttt tcg acg ggc gcg gac ttc tcg ctc aac ggc 720
Ser Asp Glu Ser Gly Phe Ser Thr Gly Ala Asp Phe Ser Leu Asn Gly
225                 230                 235                 240

ggc ctg cat atg ggc tga 738
Gly Leu His Met Gly
                245

<210> 3
<211> 246
<212> PRT
<213> Ralstonia eutropha

<400> 3
Met Thr Gln Arg Ile Ala Tyr Val Thr Gly Gly Met Gly Gly Ile Gly
1               5                   10                  15

Thr Ala Ile Cys Gln Arg Leu Ala Lys Asp Gly Phe Arg Val Val Ala
            20                  25                  30

Gly Cys Gly Pro Asn Ser Pro Arg Arg Glu Lys Trp Leu Glu Gln Gln

35                  40                  45

Lys Ala Leu Gly Phe Asp Phe Ile Ala Ser Glu Gly Asn Val Ala Asp
    50                  55                  60

Trp Asp Ser Thr Lys Thr Ala Phe Asp Lys Val Lys Ser Glu Val Gly
65                  70                  75                  80

Glu Val Asp Val Leu Ile Asn Asn Ala Gly Ile Thr Arg Asp Val Val
                85                  90                  95

Phe Arg Lys Met Thr Arg Ala Asp Trp Asp Ala Val Ile Asp Thr Asn
            100                 105                 110

Leu Thr Ser Leu Phe Asn Val Thr Lys Gln Val Ile Asp Gly Met Ala
        115                 120                 125

Asp Arg Gly Trp Gly Arg Ile Val Asn Ile Ser Ser Val Asn Gly Gln
    130                 135                 140

Lys Gly Gln Phe Gly Gln Thr Asn Tyr Ser Thr Ala Lys Ala Gly Leu
145                 150                 155                 160

His Gly Phe Thr Met Ala Leu Ala Gln Glu Val Ala Thr Lys Gly Val
                165                 170                 175

Thr Val Asn Thr Val Ser Pro Gly Tyr Ile Ala Thr Asp Met Val Lys
            180                 185                 190

Ala Ile Arg Gln Asp Val Leu Asp Lys Ile Val Ala Thr Ile Pro Val
        195                 200                 205

Lys Arg Leu Gly Leu Pro Glu Glu Ile Ala Ser Ile Cys Ala Trp Leu
    210                 215                 220

Ser Ser Glu Glu Ser Gly Phe Ser Thr Gly Ala Asp Phe Ser Leu Asn
225                 230                 235                 240

Gly Gly Leu His Met Gly
                245


<210>  4
<211>  741
<212>  DNA
<213>  Ralstonia eutropha

<220>
<221>  CDS
<222>  (1)..(741)
<223>

<400>  4
atg act cag cgc att gcg tat gtg acc ggc ggc atg ggt ggt atc gga      48
Met Thr Gln Arg Ile Ala Tyr Val Thr Gly Gly Met Gly Gly Ile Gly
1               5                   10                  15

acc gcc att tgc cag cgg ctg gcc aag gat ggc ttt cgt gtg gtg gcc      96
Thr Ala Ile Cys Gln Arg Leu Ala Lys Asp Gly Phe Arg Val Val Ala
            20                  25                  30

ggt tgc ggc ccc aac tcg ccg cgc cgc gaa aag tgg ctg gag cag cag      144
Gly Cys Gly Pro Asn Ser Pro Arg Arg Glu Lys Trp Leu Glu Gln Gln
        35                  40                  45

aag gcc ctg ggc ttc gat ttc att gcc tcg gaa ggc aat gtg gct gac      192
Lys Ala Leu Gly Phe Asp Phe Ile Ala Ser Glu Gly Asn Val Ala Asp
    50                  55                  60

tgg gac tcg acc aag acc gca ttc gac aag gtc aag tcc gag gtc ggc      240
Trp Asp Ser Thr Lys Thr Ala Phe Asp Lys Val Lys Ser Glu Val Gly
65                  70                  75                  80

gag gtt gat gtg ctg atc aac aac gcc ggt atc acc cgc gac gtg gtg      288
Glu Val Asp Val Leu Ile Asn Asn Ala Gly Ile Thr Arg Asp Val Val
                85                  90                  95

ttc cgc aag atg acc cgc gcc gac tgg gat gcg gtg atc gac acc aac      336
Phe Arg Lys Met Thr Arg Ala Asp Trp Asp Ala Val Ile Asp Thr Asn
            100                 105                 110

ctg acc tcg ctg ttc aac gtc acc aag cag gtg atc gac ggc atg gcc 384
Leu Thr Ser Leu Phe Asn Val Thr Lys Gln Val Ile Asp Gly Met Ala
        115                 120                 125

gac cgt ggc tgg ggc cgc atc gtc aac atc tcg tcg gtg aac ggg cag 432
Asp Arg Gly Trp Gly Arg Ile Val Asn Ile Ser Ser Val Asn Gly Gln
    130                 135                 140

aag ggc cag ttc ggc cag acc aac tac tcc acc gcc aag gcc ggc ctg 480
Lys Gly Gln Phe Gly Gln Thr Asn Tyr Ser Thr Ala Lys Ala Gly Leu
145                 150                 155                 160

cat ggc ttc acc atg gca ctg gcg cag gaa gtg gcg acc aag ggc gtg 528
His Gly Phe Thr Met Ala Leu Ala Gln Glu Val Ala Thr Lys Gly Val
                165                 170                 175

acc gtc aac acg gtc tct ccg ggc tat atc gcc acc gac atg gtc aag 576
Thr Val Asn Thr Val Ser Pro Gly Tyr Ile Ala Thr Asp Met Val Lys
            180                 185                 190

gcg atc cgc cag gac gtg ctc gac aag atc gtc gcg acg atc ccg gtc 624
Ala Ile Arg Gln Asp Val Leu Asp Lys Ile Val Ala Thr Ile Pro Val
        195                 200                 205

aag cgc ctg ggc ctg ccg gaa gag atc gcc tcg atc tgc gcc tgg ttg 672
Lys Arg Leu Gly Leu Pro Glu Glu Ile Ala Ser Ile Cys Ala Trp Leu
    210                 215                 220

tcg tcg gag gag tcc ggt ttc tcg acc ggc gcc gac ttc tcg ctc aac 720
Ser Ser Glu Glu Ser Gly Phe Ser Thr Gly Ala Asp Phe Ser Leu Asn
225                 230                 235                 240

ggc ggc ctg cat atg ggc tga 741
Gly Gly Leu His Met Gly
                245

<210> 5
<211> 19
<212> DNA
<213> Artificial Sequence

<220>
<223> Primer 1

<220>
<221> misc_feature
<222> (6)..(6)
<223> n represents a, t, g or c

<220>
<221> misc_feature
<222> (12)..(12)
<223> n represents a, t, g or c

<400> 5
carggntaya cnttytayg 19

<210> 6
<211> 20
<212> DNA
<213> Artificial Sequence

<220>
<223> Primer 2

<220>
<221> misc_feature
<222> (12)..(12)
<223> n represents a, t, g or c

<220>
<221> misc_feature
<222> (15)..(15)
<223> n represents a, t, g or c

<400> 6
gcdatytcyt cnggngtycc 20

<210>  7
<211>  25
<212>  DNA
<213>  Artificial Sequence

<220>
<223>  Primer 3

<400>  7
cgtcggcgct catcttgcgg aacag                                                25


<210>  8
<211>  25
<212>  DNA
<213>  Artificial Sequence

<220>
<223>  Primer 4

<400>  8
agggcatcac ggtcaacacg gtgtc                                                25


<210>  9
<211>  28
<212>  DNA
<213>  Artificial Sequence

<220>
<223>  Primer 5

<400>  9
gtacatatga gcggaaaact ggcttacg                                             28


<210>  10
<211>  34
<212>  DNA
<213>  Artificial Sequence

<220>
<223>  Primer 6

<400>  10
gtagaattct tatcagccca tgtgcaggcc gccg                                      34


<210>  11
<211>  43
<212>  DNA
<213>  Artificial Sequence

<220>
<223>  Primer 7

<400>  11
gccgaattct aaggaggtta acaatgtata aagatttaga agg                            43


<210>  12
<211>  28
<212>  DNA
<213>  Artificial Sequence

<220>
<223>  Primer 8

<400>  12
gcggtcgact tatccgcgtc ctgcttgg                                             28


<210>  13
<211>  35
<212>  DNA
<213>  Artificial Sequence

<220>

<223>  Primer 9

<400>  13
aaggagtgca tatgactcag cgcattgcgt atgtg                                35


<210>  14
<211>  34
<212>  DNA
<213>  Artificial Sequence

<220>
<223>  Primer 10

<400>  14
gtagaattct tatcagccca tgtgcaggcc gccg                                 34


<210>  15
<211>  900
<212>  DNA
<213>  Achromobacter xylosoxidans subsp. denitrificans

<220>
<221>  CDS
<222>  (40)..(778)
<223>

<400>  15
tcaatcaacg aggcgtccgt cgcacacagg aggaaatcca atgagcggaa aactggctta     60

cgttacaggc gggatgggcg gtatcggcac ctcaatttgc cagcgcctgg ccaaagatgg    120

ctttcgcgtg gtggcaggct gcggccccag ccgcaattac cagcaatggc tggatgaaca    180

ggcggcgcag ggctatacgt tctacgcgtc agtgggcaac gtgtccgatt gggagtccac    240

ggtagaagca ttcgagcgcg tcaagcggga catgggcccg gtcgatgtgc tggtcaacaa    300

cgcgggcatc acccgcgacg gcctgttccg caagatgagc gccgacgact ggcgcgcggt    360

catcgacacc aacctgaaca gcctcttcaa cgtgaccaag caggtgatcg acgacatggt    420

cgagcgccag tggggccgca tcgtcaacat cagctcggtg aacgggcaga aggggcagtt    480

cggccagacg aactattcca cggcgaaggc gggcatccat ggcttcacca tggcgctggc    540

gcaggaagtg gccagcaagg gcatcacggt caacacggtg tcgccgggct acatcggcac    600

ggacatggtt cgcgccatcc gtccggacgt gctggaaaag atcgtcgcca ccattccggt    660

gcgccgcctg ggcacgccgg aggaaatcgc gtccatcacg tcgtggctgg cctcggatga    720

gtctgggttt tcgacgggcg cggacttctc gctcaacggc ggcctgcata tgggctgaag    780

catcgcgggc cgccacgagc ggcccgccgg cgccgggcgg cctcggggag agggccgtcc    840

ggcattacac ttacccttat ccgaagtctt agagatcgcc cgatccgggg acaaccatga    900